(19) Europäisches Patentamt
European Patent Office
Office européen des brevets

(11) Veröffentlichungsnummer: **0 370 198**
**A1**

(12) # EUROPÄISCHE PATENTANMELDUNG

(21) Anmeldenummer: **89117618.2**

(22) Anmeldetag: **23.09.89**

(51) Int. Cl.5: **C07D 209/10, C07D 403/06, B41M 5/124**

(30) Priorität: **06.10.88 DE 3833997**

(43) Veröffentlichungstag der Anmeldung:
**30.05.90 Patentblatt 90/22**

(84) Benannte Vertragsstaaten:
**CH DE FR GB IT LI NL**

(71) Anmelder: **BAYER AG**
**D-5090 Leverkusen 1 Bayerwerk(DE)**

(72) Erfinder: **Berneth, Horst, Dr.**
**Erfurther Strasse 1**
**D-5090 Leverkusen 1(DE)**
Erfinder: **Psaar, Hubertus, Dr.**
**Paul-Klee-Strasse 21**
**D-5090 Leverkusen 1(DE)**
Erfinder: **Klug, Günter, Dr.**
**Wiener-Neustaedter-Strasse 140**
**D-4019 Monheim 2(DE)**

(54) **Tetraindolyl-heptamethin-derivate.**

(57) Tetraindolyl-heptamethin-derivate der isomeren Formeln

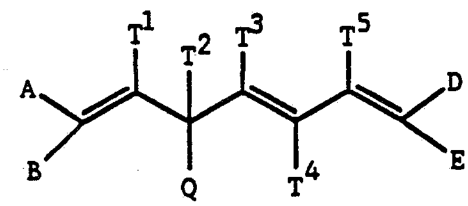

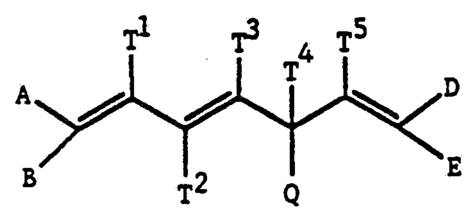

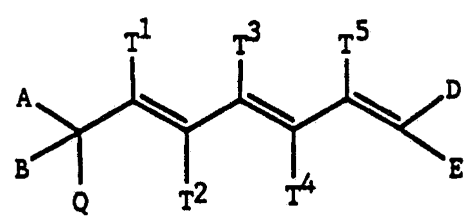

und

EP 0 370 198 A1

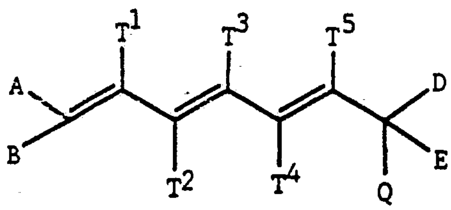

worin A, B, D und E

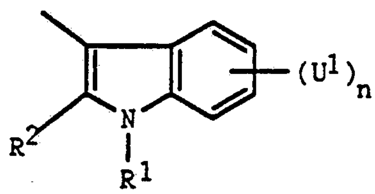

bedeuten und untereinander gleich oder verschieden sein können, und die übrigen Symbole die in der Beschreibung angegebenen Bedeutung haben, finden Verwendung in druck-und thermoempfindlichen aufzeichnungsmaterialien.

## Tetraindolyl-heptamethin-derivate

Gegenstand der Erfindung sind Tetraindolyl-heptamethinderivate der isomeren Formeln

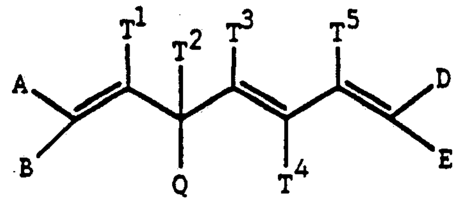
(I),

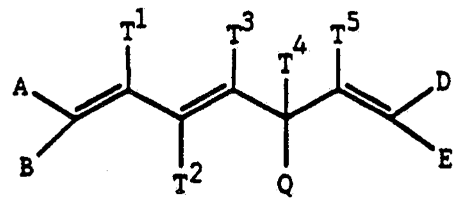
(II),

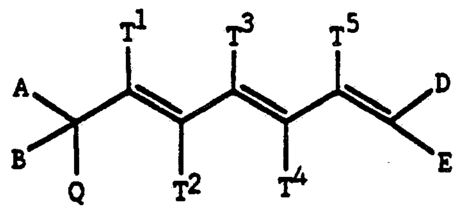
(III)

und

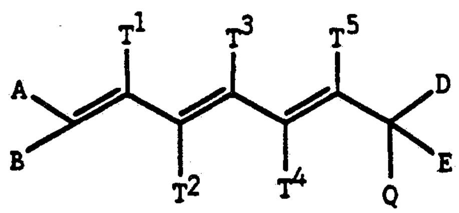
(IV),

worin A, B, D und E

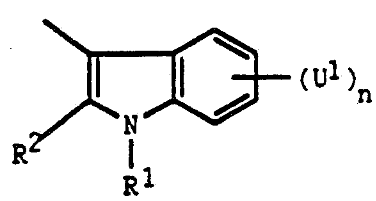

bedeuten und untereinander gleich oder verschieden sein können,
Q $NV^1V^2$, SW, $SO_2X$ oder $CLY^1Y^2$,
$R^1$ Wasserstoff, Alkyl, Alkenyl, Cycloalkyl, Aralkyl oder ein über Alkyl gebundener heterocyclischer Rest,
$R^2$ Wasserstoff, Alkyl, Alkenyl, Cycloalkyl, Aralkyl, Aryl oder ein gegebenenfalls über Alkyl gebundener heterocyclischer Rest,

$T^1$ bis $T^5$ Wasserstoff, Alkyl, Alkenyl, Cycloalkyl, Aralkyl, Halogen, Alkoxy, Dialkylamino, Cyano, Hydroxycarbonyl, Alkoxycarbonyl, Aryl, ein ggf. über Alkyl gebundener heterocyclischer Rest oder jeweils zwei der Reste $T^1$ bis $T^5$ die fehlenden Glieder eines fünf- bis sieben-gliedrigen Ringes, der aromatisch oder teilhydriert sein und bis zu 2 Heteroatome aus der Reihe O, N oder S enthalten kann,

$U^1$ Wasserstoff, Alkyl, Alkenyl, Cycloalkyl, Aralkyl, Aryl, Hydroxy, Alkoxy, Halogen, Dialkylamino, Nitro, Cyano, Alkylthio, Alkoxycarbonyl, Dialkylaminocarbonyl, Alkoxycarbonyloxy oder Alkylsulfonyl oder zusammen mit $R^1$ eine $C_2$- oder $C_3$-Brücke,

$V^1$ und $V^2$ unabhängig voneinander Wasserstoff, Alkyl, Alkenyl, Cycloalkyl, ggf. über Alkyl gebundene Aryl- oder heterocyclische Reste, $-CO-Z^1$,

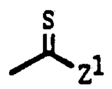 , 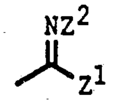 ,

CN oder gemeinsam mit dem Stickstoff einen ggf. vollständig oder teilweise hydrierten, 0-3 Heteroatome der Reihe O, N, S, $SO_2$ enthaltenden Ring,

W Wasserstoff, Alkyl, Alkenyl, Cycloalkyl, ggf. über Alkyl gebundene Aryl- oder heterocyclische Reste,

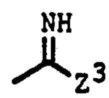

oder CN,

X Alkyl, Alkenyl, Cycloalkyl oder ggf. über Alkyl gebundene Aryl- oder heterocyclische Reste,

L Wasserstoff, Alkyl, Alkenyl, Cycloalkyl, Halogen, über Alkyl gebundene Aryl- oder heterocyclische Reste, oder die Bedeutung von $Y^1$ bzw. $Y^2$ hat,

$Y^1$ und $Y^2$ $-CO-Z^1$,

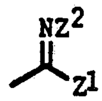 ,

$-CN$, $-SO_2Z^1$, $NO_2$, Aryl oder ein heterocyclischer Rest,

$Z^1$ bis $Z^3$ Wasserstoff, Alkyl, Alkenyl, Cycloalkyl, Alkoxy, Alkylthio, Cycloalkoxy, Amino, Mono-oder Bisalkylamino, Arylamino oder ggf. über Alkyl oder Alkoxy gebunden Aryl- oder heterocyclische Reste oder

$Z^1$ und $Z^2$ mit $V^1$ oder $V^2$ bzw. $Y^1$ oder $Y^2$ oder einem anderen Rest $Z^{1'}$ oder $Z^{2'}$ die fehlenden Glieder eines ggf. vollständig oder teilweise hydrierten, 0-2 Heteroatome der Reihe O, N, S oder $SO_2$ enthaltenden, ggf. benzanellierten Ringes, wobei

$Z^{1'}$ und $Z^{2'}$ die Bedeutung von $Z^1$ bzw. $Z^2$ besitzen und

n 1 oder 2 bedeuten

und alle cyclischen und nichtcyclischen Reste in der Farbstoffchemie übliche nichtionische Substituenten tragen können, ihre Herstellung und Verwendung in druck-und thermoempfindlichen Aufzeichnungsmaterialien, die im Infrarotbereich 750-950 nm gelesen werden können, sowie Aufzeichnungsmaterialien, die diese Verbindungen enthalten.

Alkylreste, auch solche in z.B. Alkoxy, Alkylamino oder Aralkyl, können bis zu 18 C-Atome aufweisen und z.B. durch Halogen, Alkoxy, Nitro, Cyano, Alkoxycarbonyl oder Alkylsulfonyl substituiert sein.

Alkenylreste können bis zu 18 C-Atome aufweisen und z.B. durch Halogen, Alkoxy, Cyano oder Alkoxycarbonyl substituiert sein.

Cycloalkylreste können 3-8 C-Atome aufweisen und z.B. durch Alkyl, Alkoxy, Halogen, Cyano, Alkoxycaronyl oder Aryl substituiert sein.

Arylreste, auch solche in Aralkylgruppen, sind Phenyl, Naphthyl oder Anthracenyl, die z.B. durch Alkyl, Alkoxy, Halogen, Cyano, Alkoxycarbonyl, Nitro, Aryl oder heterocyclische Reste substituiert sein können, wobei bis zu 5 Substituenten, die nicht gleich sein müssen, möglich sind.

Heterocyclische Reste, auch solche, die über Alkyl gebunden sind, fünf- bis siebengliedrige aromatische oder quasiaromatische Heterocyclen oder ihre teilweise oder vollständig hydrierten Derivate, die O, N,

S oder $SO_2$ als Heteroatome enthalten, wobei maximal 4 solcher Heteroatome, die auch untereinander gemischt sein können in einem Ring auftreten, und wobei diese Heterocyclen durch Benzol, Naphthalin oder Pyridin anelliert und/oder durch Alkyl, Alkoxy, Halogen, Cyano, Alkoxycarbonyl, Ni tro oder Aryl substituiert sein können.

Bevorzugt sind Tetraindolylheptamethinderivate der isorenen Formeln

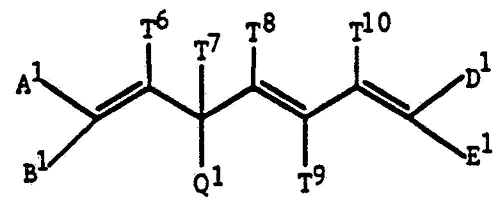
(V),

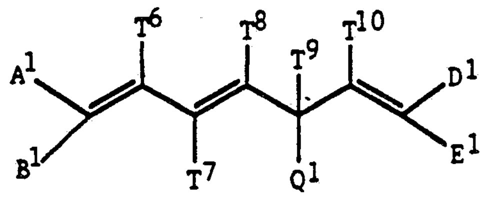
(VI),

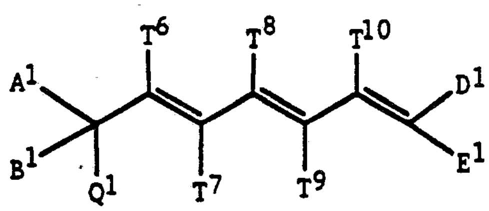
(VII)

und

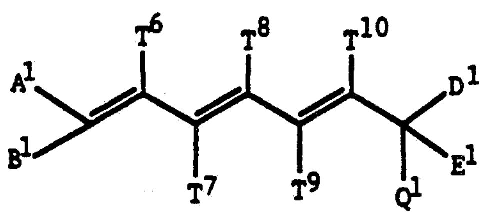
(VIII),

worin $A^1$, $B^1$, $D^1$ und $E^1$

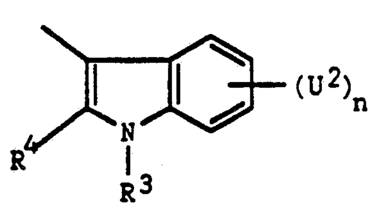

bedeuten und untereinander gleich oder verschieden sein können,

Q¹ $-NV^3V^4$, 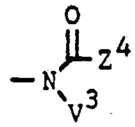 , 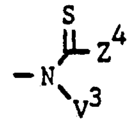 , 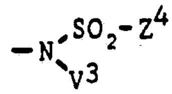 ,

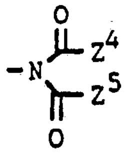 , 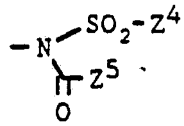 , 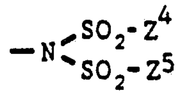 , $-SW^1$,

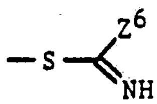 , $-SO_2X^1$, 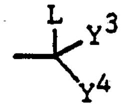 , 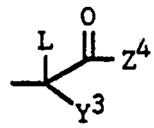 ,

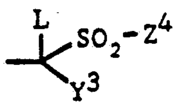 , 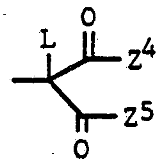 , 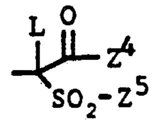 , 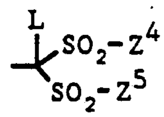 ,

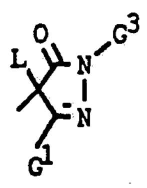 , 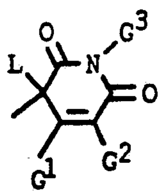 ,

$R^3$ Wasserstoff, $C_1$-$C_{18}$-Alkyl, das durch Chlor, $C_1$-CÜ-Alkoxy, Cyano oder $C_1$-$C_4$-Alkoxycarbonyl substituiert sein kann, Allyl, Cyclopentyl, Cyclohexyl, ggf. durch $C_1$-$C_4$-Alkyl, Chlor und/oder $C_1$-$C_4$-Alkoxy substituierte Benzyl-, Phenethyl-, Naphthylmethyl- oder Picolylreste,

$R^4$ Wasserstoff, $C_1$-$C_{18}$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, ggf. durch $C_1$-$C_4$-Alkyl, Chlor, Brom, $C_1$-$C_4$-Alkoxy, Cyano, Nitro und/oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl- oder chinolylreste,

$T^6$-$T^{10}$ Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Vinyl, allyl, Cyclopentyl, Cyclohexyl, Fluor, Chlor, Brom, $C_1$-$C_8$-Alkoxy, das noch durch $C_1$-$C_4$-Alkoxy substituiert sein kann, $C_1$-$C_4$-Dialkylamino, Piperidino, Pyrrolidino, Nitro, Cyano, $C_1$-$C_4$-Alkoxycarbonyl, ggf. durch $C_1$-$C_4$-Alkyl, Cyclor, $C_1$-$C_4$-Alkoxy, $C_1$-$C_4$-Alkylsulfonyl, Cyano und/oder $C_1$-$C_4$-Alkoxycarbonyl substituierte Benzyl-, Phenethyl-, Naphthylmethyl-, Picolyl-, Phenyl-, Naphthyl-, Pyridyl-, Chinolyl-, Pyrimidyl-, Pyrazinyl-, Indolyl-, Indolenyl-, Indolizinyl-, Imidazolyl-, Oxazolyl-, Thiazolyl-, Triazolyl-, Benzimidazolyl-, Benzoxazolyl- oder Benzthiazolylreste oder jeweils zwei der Reste $T^6$ bis $T^{10}$ eine Brücke der For meln $-CH_2CH_2-$, $-CH_2CH_2CH_2-$, $-CH_2CH_2CH_2CH_2-$, $-CH_2SCH_2-$,

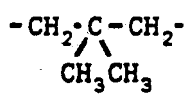 , 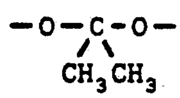 ,

$-CH=CH-CH_2-$, $-CH=CH-CH=CH-$,

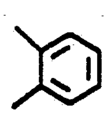 , 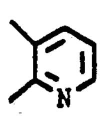 , 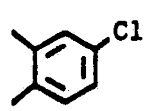 ,

$U^2$ Wasserstoff, $C_1$-$C_8$-Alkyl, Allyl, Cyclohexyl, Benzyl, Phenyl, Hydroxy, $C_1$-$C_4$-Alkoxy, Chlor, Brom, $C_1$-$C_4$-Dialkylamino, Nitro, Cyano, $C_1$-$C_4$-Alkylthio, $C_1$-$C_4$-Alkoxycarbonyl, $C_1$-$C_4$-Dialkylaminocarbonyl, $C_1$-$C_4$-Alkoxycarbonyloxy oder $C_1$-$C_4$-Alkylsulfonyl oder zusammen mit $R^3$ eine $-CH_2CH_2-$ oder $-CH_2CH_2CH_2-$Brücke, die durch maximal drei Methylgruppen substituiert sein kann,

$V^3$, $V^4$ und $W^1$ unabhängig voneinander Wasserstoff, $C_1$-$C_{18}$-Alkyl, das durch Chlor, $C_1$-$C_4$-Alkoxy, Cyano oder $C_1$-$C_4$-Alkoxy, Cyano, Nitro oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl-, Chinolyl-, Tetrahydrothiophendioxid-3-yl-, Piperidin-4-yl- oder Piperazin-1-yl-ethyl- Reste oder

$V^3$ und $V^4$ zusammen mit dem Stickstoff ggf. durch $C_1$-$C_4$-Alkyl, Chlor, $C_1$-$C_4$-Alkoxy oder Phenyl substituierte Pyrrolidin-, Piperidin-, Piperasin-, Morpholin-, Pyrazolin-, Pyrazol-, Imidazol-, Triazol- oder Tetrazol-Reste,

$X^1$ $C_1$-$C_{18}$-Alkyl, das durch Chlor, $C_1$-$C_4$-Alkoxy, Cyano oder $C_1$-$C_4$-Alkoxycarbonyl, substituiert sein kann, Allyl, Cyclopentyl, Cyclohexyl, ggf. durch $C_1$-$C_4$-Alkyl, Chlor, Brom, $C_1$-$C_4$-Alkoxy, Cyano, Nitro oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimdyl-, Pyrazinyl-, Imidazolyl-, Oxazolyl-, Thiazolyl-, Triazolyl-, Benzimidazolyl-, Benzoxazolyl-, Benzthiazolyl- oder Chinolyl-Reste,

L Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, Chlor, Cyano, $C_1$-$C_8$- Alkoxycarbonyl, ggf. durch $C_1$-$C_4$-Alkyl, Chlor, $C_1$-$C_4$-Alkoxy, Cyano, Nitro oder $C_1$-$C_4$-Alkoxycarbonyl substituierte Benzyl-, Phenethyl-, Picolyl-, Phenyl-, Naphthyl- oder Pyridyl-Reste,

$Y^3$ und $Y^4$ Cyano, Nitro oder ggf. durch $C_1$-$C_4$-Alkyl, Chlor, Brom, $C_1$-$C_4$-Alkoxy, Cyano, Nitro, $C_1$-$C_4$-Alkylsulfonyl oder $C_1$-$C_4$-Alkoxycarbonyl substituierte Phenyl-, Naphthyl-, Pyridyl-, Pyrimidyl-, Pyrazinyl-, Imidazolyl-, Oxazo lyl-, Thiazolyl-, Triazolyl-, Benzimidazolyl-, Benzoxazolyl-, Benzthiazolyl- oder Chinolyl-Reste,

$Z^4$,$Z^5$,$Z^6$ unabhängig voneinander Wasserstoff, $C_1$-$C_{18}$-Alkyl, das durch chlor, $C_1$-$C_4$-Alkoxy, Cyano oder $C_1$-$C_4$-Alkoxycarbonyl substituiert sein kann, Allyl, Cyclopentyl, Cyclohexyl, $C_1$-$C_{18}$-Alkoxy, $C_1$-$C_{18}$-Alkylthio, Cyclopentoxy, Cyclohexoxy, Amino, mono- oder bis-$C_1$-$C_{12}$-Alkylamino, ggf. durch $C_1$-$C_4$-Alkyl, Chlor, Brom, $C_1$-$C_4$-Alkoxy, Cyano, Nitro oder $C_1$- $C_4$-Alkoxycarbonyl substituierte Anilino-, N-$C_1$-$C_4$-Alkylanilino-, Diphenylamino-, Benzyl-, Benzylxy-, Benzylamino-, N-$C_1$-$C_4$-Alkylbenzylamino-, Dibenzylamino-, Phenethyl-, Phenethoxy-, Phenethamino-, Pyridylethoxy-, Pyridylamino-, Picolyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl-, Chinolyl- oder Piperazin-1-yl-ethyl-Reste oder

$Z^4$ mit $Z^5$ oder $V^3$ oder $Y^3$ eine Brücke der formeln $-Ch_2CH_2-$, $-CH=CH-$, $-CH_2CH_2CH_2-$, $-CH_2CH_2CH_2CH_2-$,

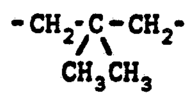 , 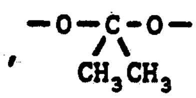 ,

$-CH=CH-CH_2-$, $-CH=CH-CH=CH-$,

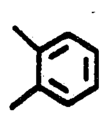 , 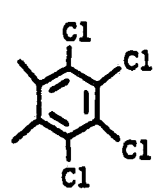 ,

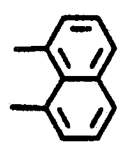 , 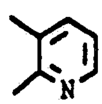 , 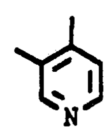 ,

$G^1$ und $G^2$ unabhängig voneinander Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, Hydroxy, Chlor, $C_1$-$C_8$-Alkoxy,

Cyano, $C_1$-$C_8$-Alkoxycarbonyl, Nitro, $C_1$-$C_8$-Alkoxy, Cyano, Nitro oder $C_1$-$C_4$-Alkoxycarbonyl substituierte Benzyl-, Phenethyl-, Picolyl-, Phenyl-, Naphthyl-, Pyridyl-, Imidazolyl-, Oxazolyl-, Thiazolyl-, Triazolyl-, Benzimidazolyl-, Benzthiazolyl- oder Benzoxazolyl-Reste,

$G^3$ Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, ggf. durch $C_1$-$C_4$-Alkyl, Chlor, $C_1$-$C_4$-Alkoxy, cyano, Nitro oder $C_1$-$C_4$-Alkoxycarbonyl substituierte Benzyl-, Phenethyl-, Picolyl-, Phenyl-, Naphthyl- oder Pyridyl-Reste und

n 1 oder 2 bedeuten.

Besonders bevorzugt sind Tetraindolylheptamethinderivate der Formel V bis VIII, worin $Q^1$ $C_1$-$C_8$-Mono- oder Dialkylamino, ggf. durch Methyl, Chlor oder Methoxy substituierte Pyrrolidino-, Piperidino-, Morpholino-, Piperazino-, Hydroxyethylpiperazino-, Pyrazolo-, Imidazolo, Triazolo-, Tetrazolo-Reste, ggf. durch Methyl, Ethyl, Chlor, Cyano oder Nitro substituierte Anilino-, N-$C_1$-$C_4$-Alkylanilino-, Diphenylamino-, Benzylamino, N-$C_1$-$C_4$-Alkyl-N-benzylamino-, Pyridylamino-, Benzoylamino-, N-$C_1$-$C_4$-Alkylbenzoylamino-, N-Phenylbenzoylamino-, Benzolsulfonylamino-, N-$C_1$-$C_4$-Alkylbenzolsulfonylamino-, Phthalimido-, Naphthalimido-, Homophthalimido-, Benzoesäure-2-sulfonsäureimido-, Succinimido-, Maleinimido-Reste, Aminocarbonylamino, $C_1$-$C_8$-Alkoxycarbonylamino, $C_1$-$C_8$-Alkylthio, ggf. durch Methyl, Chlor oder Methoxy substituiertes Phenylthio, $H_2N-\underset{NH}{\underset{\|}{C}}-S-$,

$C_1$-$C_8$-Alkylsulfonyl, ggf. durch Methyl, Ethyl, Chlor, Brom, Cyano oder Methoxycarbonyl substituierte Phenylsulfonyl-, Benzylsulfonyl-, Naphthylsulfonyl-, in $\alpha$-Stellung durch Cyano, $C_1$-$C_4$-Alkoxycarbonyl, $C_1$-$C_4$-Alkylsulfonyl, $C_1$-$C_4$-Alkanoyl oder Benzoyl substituierte, ggf. weiterhin durch Methyl, Ethyl, Chlor, Brom, Cyano, Nitro oder Methoxycarbonyl substituierte Benzyl-, Naphthylmethyl-, Picolyl-, Chinolylmethyl-, Imidazolylmethyl-, Benzimidazolylmethyl-, Oxazolylmethyl-, Benzoxazolylmethyl-, Thiazolylmethyl-, Benzthiazolylmethyl-, Triazolylmethyl-, Tetrazolylmethyl-Reste, Dicyanomethyl, Cyano-$C_1$-$C_4$-Alkoxycarbonylmethyl, Bis-$C_1$-$C_4$-alkoxycarbonylmethyl, Cyano-$C_1$-$C_4$-alkanoylmethyl, Bis-$C_1$-$C_4$-alkanoylmethyl, ggf. durch Methyl, Ethyl, Chlor, Cyano, Nitro oder Methoxycarbonyl substituierte Cyano-benzoylmethyl-, $C_1$-$C_4$-Alkanoyl-benzoylmethyl-, $C_1$-$C_4$-Alkoxycarbonyl-benzoylmethyl-, Dibenzoylmethyl-, Cyano-phenylsulfonylmethyl-, $C_1$-$C_4$-Alkanoyl-phenylsulfonylmethyl-, $C_1$-$C_4$-Alkoxycarbonyl-phenylsulfonylmethyl-, BisphenylsulfonylmethylReste, die Reste von ggf. durch Methyl, Ethyl, Chlor, Methoxy, Ethoxy, Phenyl, Cyano, Methoxy- oder ethoxycarbonyl substituiertem 1,3-Cyclopentandion, 1,3-Cyclohexandion, 1,3-Indandion, Meldrumsäure, Pyrazolon, 2-Hydroxy-6-pyridon oder Barbitursäure,

$R^3$ Wasserstoff, $C_1$-$C_8$-Alkyl, das durch Chlor, Methoxy, Ethoxy, Cyano oder Methoxycarbonyl substituiert sein kann, Allyl, Cyclopentyl, Cyclohexyl, ggf. durch Methyl, Chlor oder Methoxy substituierte Benzyl-, Phenethyl- oder Picolyl-Reste,

$R^4$ Wasserstoff, $C_1$-$C_8$-Alkyl, das durch Chlor, Methoxy, Ethoxy, Cyano oder Methoxycarbonyl substituiert sein kann, Allyl, cyclopentyl, Cyclohexyl, ggf. durch Methyl, Chlor, Methoxy, Cyano, Nitro und/oder Methoxycarbonyl substituierte Benzyl-, Phenethyl-, Picolyl-, Phenyl-, Naphthyl-, Pyridyl-, Pyrimidyl-, Benz imidazolyl-, Benzoxazolyl-, Benzthiazolyl-oder Chinolyl-Rest,

$T^6$ u. $T^{10}$ Wasserstoff, $C_1$-$C_8$-Alkyl, das durch chlor, Methoxy, Cyano oder Methoxycarbonyl substituiert sein kann, Vinyl, Allyl, Cyclopentyl, Cyclohexyl, Chlor, $C_1$-$C_8$-Alkoxy, Cyano, Methoxycarbonyl, Nitro, Benzyl, ggf. durch Methyl, Chlor, cyano oder Methoxy substituierte Phenyl- oder Pyridyl-Reste,

$T^7$ bis $T^9$ Wasserstoff, $C_1$-$C_8$-Alkyl, das durch Chlor, Methoxy, Cyano oder Methoxycarbonyl substituiert sein kann, Allyl, Cyclopentyl, Cyclohexyl, Chlor, Brom, Cyano, Methoxy- oder Ethoxycarbonyl, Nitro, $C_1$-$C_4$-Alkoxy, $C_1$-$C_4$-Dialkylamino, Benzyl, ggf. durch Methyl, Ethyl, chlor, Methoxy, Ethoxy, Cyano, Nitro und/oder Methoxycarbonyl substituierte Phenyl-, Naphthyl-, Pyridyl-, Chinolyl-, Pyrimidyl-, Indolenyl, Indolizinyl-, Imidazolyl-, Oxazolyl-, Thiazolyl-, Benzimidazolyl-, Benzoxazolyl-oder Benzthiazolyl-Reste oder

$T^7$ mit $T^8$ oder $T^9$ oder $T^8$ mit $T^9$ eine Brücke der Formeln $-CH_2CH_2-$, $-CH_2CH_2CH_2-$,

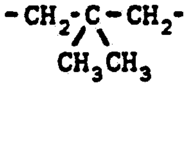

,

$-CH=CH-CH_2-$, $-CH=CH-CH=CH-$ oder

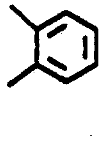

,

$U^2$ Wasserstoff, $C_1$-$C_4$-Alkyl, Cyclohexyl, Benzyl, $C_1$-$C_4$-Alkoxy, Chlor, $C_1$-$C_4$-Dialkylamino, Nitro, Cyano, Methoxy- oder Ethoxycarbonyl oder Methylsulfonyl, wobei $U^2$ in 5-, 6- und/oder 7-Postition am Indolylrest stehen kann, oder ein in 7- Position stehender Rest $U^2$ zusammen mit $R^3$ eine Brücke der Formeln $-CH_2CH_2-$,

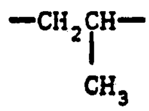 , 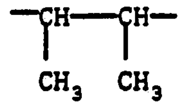 , 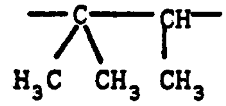 ,

$-CH_2CH_2CH_2-$,

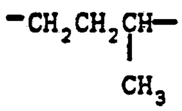 , 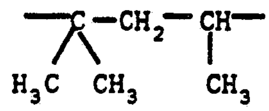

bilden kann, und n 1 oder 2 bedeuten.

Ganz besonders bevorzugt sind Tetraindolylheptamethinderivate der Formel

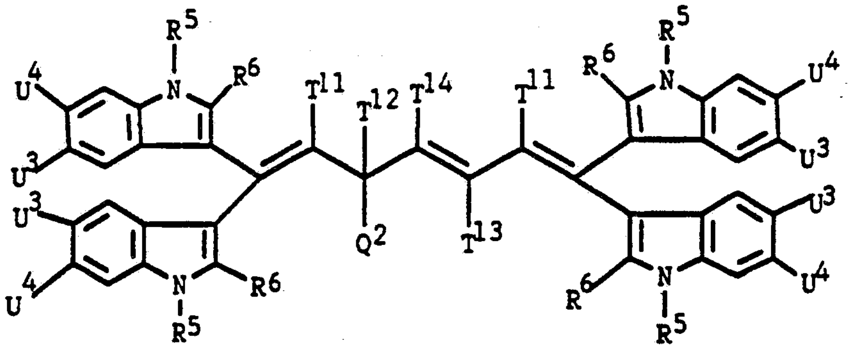

(IX)

und ihre bezüglich der Stellung der $Q^2$-Gruppe isomeren Forme, wie sie in den Formeln II bis IV und VI bis VIII wiedergegeben sind, worin

$Q^2$ Dimethylamino, Diethylamino, Dipropylamino, Dibutylamino, Propylamino, Butylamino, Pyrrolidino, Piperidino, Morpholino, Piperazino, Pyrazolo, Imidazolo, Triazolo, Anilino, 4-Methylanilino, 4-Chloranilino, 4-Methoxyanilino, 4-Nitroanilino, N-Methylanilino, Benzylamino, N-Methylbenzylamino, N-Ethylbenzylamino, Benzoylamino, Benzolsulfonylamino, Phthalimido, Naphthalimido, Succinimido, Maleinimido, Methylthio, Ethylthio, Phenylthio, Methylsulfonyl, Ethylsulfonyl, Propylsulfonyl, Butylsulfonyl, Hexylsulfonyl, Octylsulfonyl, Benzolsulfonyl, 4-Methylbenzolsulfonyl, 4-Chlorbenzolsulfonyl, α-Cyanobenzyl, α-Cyano-4-methylbenzyl, α-Cyano-4-chlorbenzyl, α-Cyano-4-nitrobenzyl, α-Cyano-α-picolyl, α-Cyano-γ-picolyl, Dicyanomethyl, Cyano-methoxycarbonylmethyl, Cyano-ethoxycarbonylmethyl, Bis-methoxycarbonylmethyl, Bis-ethoxycarbonylmethyl, 1-Cyanoaceton-1-yl, 1-Cyanobutanon-1-yl, 1-Methoxycarbonylaceton-1-yl, 1-Ethoxycarbonylaceton-1-yl, 1,3-Pentandion-3-yl, 1-Benzoylaceton-1-yl, 2-Cyano-1-phenylethanon-2-yl, 2-Methoxycarbonyl-1-phenylethanon-2-yl, Dibenzoylmethyl, Cyano-phenylsulfonylmethyl, Bisphenylsulfonylmethyl, 1,3-Cyclopentandion-2-yl, 1,3-cyclohexandion-2-yl, 5,5-Dimethyl-1,3-cyclohexandion-2-yl, 1,3-Indandion-2-yl, 2,2-Dimethylperhydro-1,3-dioxin-4,6-dion-5-yl, 3-Methyl-1-phenyl-2-pyrazolin-5-on-4-yl, 1-(2-Chlorphenyl)-3-methyl-2-pyrazolin-5-on-4-yl, 3-Methyl-1-(4-methylphenyl)-2-pyrazolin-5-on-4-yl, 3-Methyl-1-(4-nitrophenyl)-2-pyrazolin-5-on-4-yl, 3-Methyl-1-phenyl-5-imino-2-pyrazolin-4-yl, 3-Methyl-1-(3-sulfolanyl)-2-pyrazolin-5-on-4-yl, 3-Ethoxycarbonyl-1-phenyl-2-pyrazolin-5-on-4-yl, 3-Aminocarbonyl-1-phenyl-2-pyrazolin-5-on-4-yl, 4-Hydroxy-1-methyl-2(1H)-chinolinon-5-yl, 3-Cyano-1,4-dimethyl-6-hydroxy-2-(1H)-pyridon-5-yl, 2,4,6-(1H,3H,5H)-Pyrimidintrion-5-yl,

$R^5$ Methyl, ethyl, Propyl, Butyl, Hexyl, Octyl, 2-Cyanoethyl, 2-Methoxyethyl, 2-Methoxycarbonylethyl, 2-chlorethyl, 2-Acetoxyethyl, Cyclohexyl, Allyl oder Benzyl,

$R^6$ Methyl, Ethyl, Propyl, Butyl, Hexyl, Octyl, Cyclohexyl, Benzyl, Phenyl, 2-, 3- oder 4-Chlorphenyl, 2-, 3- oder 4-Methoxyphenyl, 4-Nitrophenyl, 2,4-Dichlorphenyl, 2-, 3- oder 4-Tolyl oder 2-, 3- oder 4-Pyridyl,
$T^{11}$ Wasserstoff, Methyl, Ethyl, Propyl, Butyl, Vinyl, 2-Chlorethyl, 2-Cyanoethyl, Chlor, Cyano, Phenyl, 4-Tolyl oder 4-Chlorphenyl,
$T^{12}$, $T^{13}$ Wasserstoff, Methyl, Ethyl, Propyl, Butyl, Chlor, Cyano, Methoxycaronyl, Dimethylamino, Phenyl, 4-Tolyl, 4-Chlorphenyl, Pyridyl oder $T^{12}$ und $T^{13}$ gemeinsam eine Gruppierung der Formeln $-Ch_2CH_2-$, $-CH_2CH_2CH_2-$,

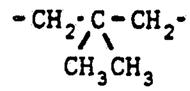 ,

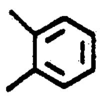 ,

$T^{14}$ Wasserstoff, Methyl, Ethyl, Propyl, Butyl, Chlor, Brom, Cyano, Phenyl, 4-Tolyl, 4-Chlorphenyl, 4-Nitrophenyl, 4-Pyridyl, 3,3-Dmethyl-indolen-2-yl, Indolizin-2-yl, 2-Benzimidazolyl, 2-Benzoxazolyl oder 2-Benzthiazolyl,
$U^3$ und $U^4$ Wasserstoff, Methyl, Methoxy, Chlor, Cyano, Methoxycarbonyl oder Nitro bedeuten.

Ebenfalls Gegenstand der Erfindung sind Verfahren zur Herstellung von Tetraindolylheptamethinderivaten der formeln I bis IV.

1. Die Herstellung erfolgt durch Kondensation von Ethenen der Formeln

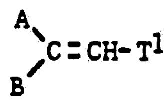 (X)

und/oder

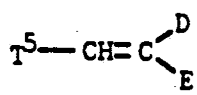 (XI)

oder Carbinolen der Formeln

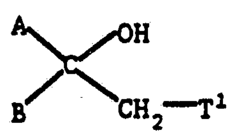 (XII)

und/oder

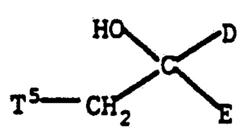 (XIII)

oder Salzen der Formeln

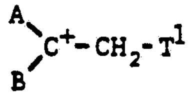 (XIV)

und/oder

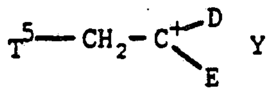 (XV)

mit 1,3-Dicarbonylverbindungen der Formel

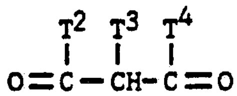 (XVI)

oder deren Acetalen oder Ketalen der Formel

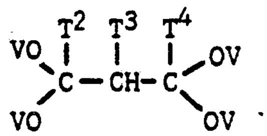 (XVII)

oder vinylogen Amidiniumsalzen der Formel

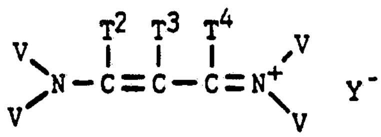 (XVIII)

oder vinylogen Chlorimmoniumsalzen der Formeln

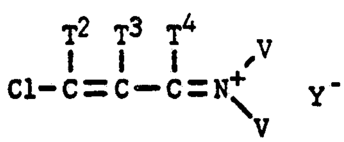 (XIX)

oder

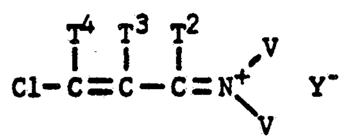 (XX)

und Umsetzung mit Verbindungen der Formel
HQ (XXI),
worin
A, B, D, E, Q und $T^1$ bis $T^5$ die oben angegbene Bedeutung besitzen und
V für Alkyl, Aralkyl oder Aryl und
$Y^-$ für ein Anion stehen.

Die Kondensation erfolgt üblicherweise im Lösungsmittel unter sauren und/oder basischen Bedingungen, wobei ein wasser- oder alkoholentziehendes Mittel zugesetzt sein kann, bei Temperaturen zwischen Raumtemperatur und dem Siedepunkt des Mediums, vorzugsweise bei 40-140 °C.

Geeignete Lösungsmittel sind Alkohole, wie Methanol, Ethanol, 1-Propanol, 2-Propanol, 1-Butanol, 1-Octanol, Cyclohexanol, Benzylalkohol; oder Ester, wie Essigsäuremethyl, -ethyl- oder -butylester; chlorierte Kohlenwasserstoffe, wie Methylenchlorid, Ethylenchlorid, Chloroform; Ketone, wie Aceton, 2-Butanon; Aromaten, wie Benzol, Toluol, Xylol; chlorierte Aromaten, wie Chlorbenzol, Dichlorbenzol; Carbonsäuren, wie Ameisensäure, Essigsäure, Propionsäure; Nitrile, wie Acetonitril oder Propionitril; und Anhydride, wie Acetanhydrid.

Geeignete Säure sind anorganische Säuren, wie Salzsäure, Schwefelsäure, Tetrafluorborsäure, Perchlorsäure, Phosphorsäure; Carbonsäuren, wie Ameisensäure, Essigsäure, Trifluoressigsäure; Sulfonsäuren, wie Methansulfonsäure, Ethansulfonsäure, Trifluormethansulfonsäure, Nonafluorbutansulfonsäure, Benzolsulfonsäure, Toluolsulfonsäure, Phosphonsäuren, wie Methanphosphonsäure; oder Ionenaustauscher, wie solche auf Basis sulfonierter Styrol-/Divinylbenzol-Polymerisate.

Geeignete Basen sind Amine, wie Triethylamin, Triethanolamin, Piperidin, Pyrrolidin oder Pyridin.

Geeignete wasser- oder alkoholentziehende Mittel sind Anhydride, wie Acetanhydrid, Trifluoressigsäureanhydrid, Benzoesäureanhydrid; Säurechloride, wie Acetylchlorid, Phosphoroxychlorid, Thionylchlorid, Oxalylchlorid, Phosgen; oder anorganische Oxide, wie Phosphorpentoxid.

Beispiel für Verbindungen der Formel XVI sind:

$O{=}CH{-}CH_2{-}CHO$, 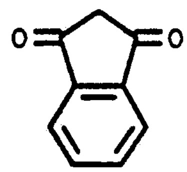 , 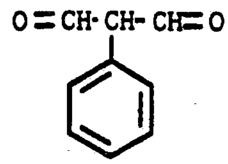 ,

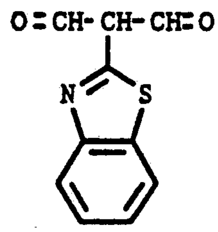 , 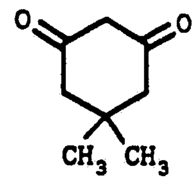 , 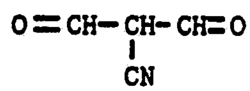 .

Beispiele für Verbindungen der Formel XVII sind
$(CH_3O)_2CH{-}CH_2{-}CH(OCH_3)_2$,

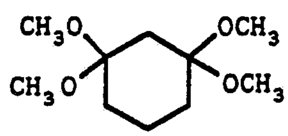 .

Beispiele für Verbindungen der Formel XVIII sind:

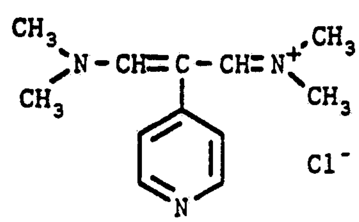

Beispiele für Verbindungen der Formel XIX sind:

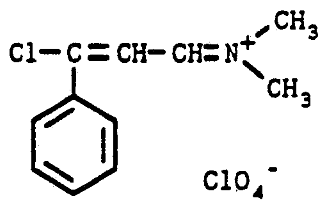
,
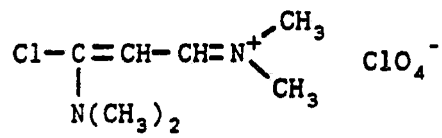

Zur Herstellung der Derivate der Formeln I-IV wird diese Kondensationsmischung entweder direkt weiterverwendet oder mit Lösungsmitteln verdünnt. Nach Zusatz der Verbindung XXI wird eine Base zugesetzt. Die Temperaturen liegen zwischen Raumtemperatur und dem Siedepunkt des Mediums, vorzugsweise zwischen 20 und 140 °C.

Zur Verdünnung geeignete Lösungsmittel sind Alkohole, Ester, Ketone oder Nitrile, wie sie weiter oben näher beschrieben sind.

Für die Umsetzung mit XXI geeignete Basen sind Amine, wie Triethylan, Triethanolamin, Piperidin, Pyrrolidin oder Pyridin; Hydroxide, wie Lithiumhydroxid, Natriumhydroxid, Kaliumhydroxid, Calciumhydroxid, Tetrabutylammoniumhydroxid; Carbonate, wie Lithiumcarbonat, Natriumcarbonat, Kaliumcarbonat, Calciumcarbonat; oder Alkoholate, wie Lithiummethylat, Natriummethylat, Natriumethylat, Natriumpropylat, Kaliummethylat, Kaliumethylat, Kalium=tert.-butylat; oder Ionenaustauscher, wie solche auf Basis aminomethylierter Styroldivinylbenzol-Polymerisate.

2. Die Herstellung erfolgt durch Umsetzung von Tetraindolylheptamethinethern oder Alkoholen der isomeren Formeln

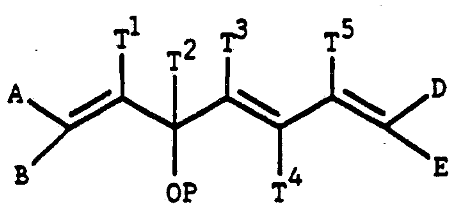
(XXII),

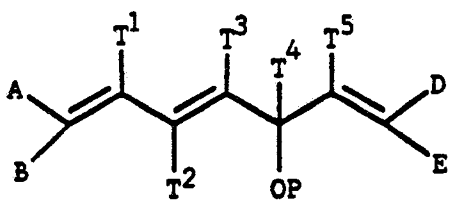
(XXIII),

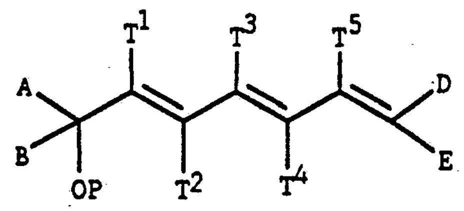

(XXIV),

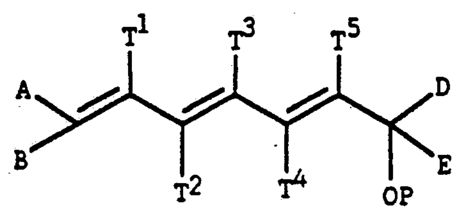

(XXV)

mit HQ (XXI), worin
A, B, D, E, Q und $T^1$ bis $T^5$ die oben angegebene Bedeutung besitzen und
P Wasserstoff, alkyl, Alkenyl, Cycloalkyl, Aralkyl, Aryl oder einen über Alkyl gebundenen heterocyclischen Rest bedeutet.

Diese Umsetzung erfolgt entweder einstufig in Gegenwart oder Abwesenheit eines Lösungsmittels und in Gegenwart oder Abwesenheit eines sauren Katalysators oder zweistufig, indem die Ether oder Alkohole der Formeln XXII-XXV mit einer Säure zu den Farbstoffen der Formel

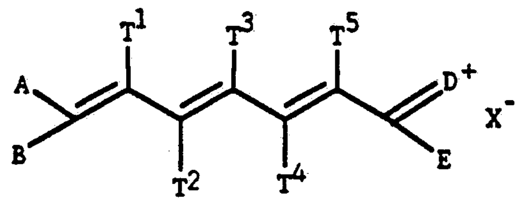

(XXVI),

worin
$D^+$ für

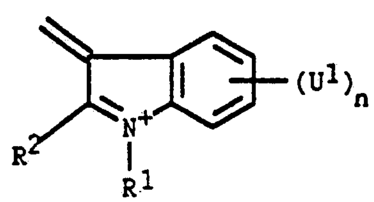

steht, A, B, E, $T^1$ bis $T^5$, $R^1$, $R^2$, $U^1$ und n die oben angegebene Bedeutung haben und
$X^-$ für ein Anion steht,
umgesetzt werden und diese Farbstoffe vorzugsweise ohne Zwischenisolierung mit HQ (XXI) und einer Base umgesetzt werden.

Geeignete Lösungsmittel sind Alkohole, Ester, chlorierte Kohlenwasserstoffe, Ketone, Aromaten, chlorierte Aromaten und Nitrile, wie sie oben unter 1. beschrieben sind.

Geeignete Säuren sind anorganische Säuren, Carbonsäuren, Sulfonsäuren oder Phosphonsäuren, wie sie oben unter 1. beschrieben sind.

Geeignete Basen sind Amine, Hydroxide, Carbonate oder Alkoholate, wie sie oben unter 1. beschrieben sind.

Gegenstand der Erfindung sind außerdem Mischungen der Verbindungen I bis IV. Die Substituenten A, B, D, E und $T^1$ bis $T^5$ können untereinander gleich oder verschieden sein. Die Mischungen können durch Mischen der Komponenten erhalten werden. Bevorzugt sind Mischungen, die direkt bei dem Verfahren 1.

erhalten werden. Si können in Abhängigkeit von den Substituenten der Ausgangskonponenten und deren Gemischen mehrere Isomere enthalten.

Solche Mischungen zeichnen sich in der Regel durch besonders gute Löslichkeit in den bei der Anwendung gebräuchlichen Lösungsmitteln aus.

Die Tetraindolylheptamethinderivate der Formeln I bis IV und ihre Mischungen sind farblose bis bräunlich gefärbte Feststoffe.

Losungen in Essigsäure zeigen eine starke Absorption mit einem Maximum im Bereich von 750-950 nm. Zusätzlich wird eine sehr schwache Absorption mit einem Maximum im Bereich 500-650 nm gefunden.

Wird eine toluolische Lösung, die farblos bis beigefarben ist, mit saurem Ton oder Kieselgel in Kontakt gebracht, so entwickelt sich spontan eine bläuliche bis grünlich graue Färbung. Zusätzlich kann im nahen Infrarotbereich von 750-950 nm eine sehr starke Absorption gemessen werden, z.B. mit Dr. Lange Farbmeßsystem Xenocolor LS 100.

Wird eine Probe eines Derivates der Formeln I bis IV mit 2,2-Bis-(4-hydroxy-phenyl)-propan vermahlen, so erhält man ein farbloses bis beigefarbenes Pulver. Erhitzt man dieses Pulver z.B. in einem Schmelzpunktröhrchen, so wird ab ca. 100° C eine kräftige schwarzblaue Farbe entwickelt.

Die Tetraindolylheptamethinderivate der Formeln I biz IV eignen sich demnach erfindungsgmäß in hervorragender Weise für druck- oder thermoempfindliche Aufzeichnungsmaterialien, die im Infrarotbereich von 750-950 nm lesbar sind.

Aufzeichnungsmaterialien, die im nahen Infrarot absorbieren, werden benötigt, um die aufgezeichnete Information mit geeigneten Geräten lesen zu können. Die Verbreitung von Computern und die automatische Datenverarbeitung erfordern Geräte, die Informationen aus Dokumenten lesen können. Es wurden deshalb Einrichtungen zur optischen Buchstabenerkennung (OCR) entwickelt, die Textseiten lesen können, die in dem jeweiligen einprogrammierten Schrifttyp geschrieben sind. Üblicherweise arbeiten solche Einrichtungen im nahen Infrarot und deshalb muß natürlich die zu lesende Schrift Absorptionen im nahen Infrarot besitzen. Übliche druck- und thermoempfindliche aufzeichnungsmaterialien weisen jedoch eine solche Absorption im nahen Infrarot nicht auf.

Aufzeichnungsmaterialien, die eine solche Absorption im nahen Infrarot besitzen sind z.B. in den US-Patentschriften 4 020 056, 4 022 771, 4 026 883, 4 107 428 und 4 119 776 und in der europäischen Anmeldung 0 124 377 beschrieben.

Die erifndungsgemäßen Tetraindolylheptamethinderivate der Formeln I bis IV und ihre Mischunge eignen sich nun in hervorragender Weise für solche OCR-lesbare aufzeichnungsmaterialien. Hierfür ist neben ihrer starken Absorption im Bereichvon 750-950 nm insbesondere die geringe Absorption im sichtbaren Spektralbereich besonders vorteilhat, da deshalb die erifndungsgemäßen Verbindungen in einfacher Weise bestehenden Farbbildnermischungen zugesetzt werden können, die in bekannter Weise z.B. blaue oder schwarze Farbtöne entwickeln, ohne daß dieser Farbton von dem entwicklungsfarbton der erfindungsgemäßen Verbindungen nennenswert beeinflußt wird.

Geeignete Farbbildner, mit denen die erfindungsgemäßen Tetrainolylheptamethinderivate der Formel I bis IV gemischt werden können, entstammen beispielsweise den Substanzklassen der Phthalide, Fluorane, Spirodipyrane, Chromenoindole, Phenoxazine, Phenothiazine, Carbazolylmethane, Dihydrochinazolone, Dihyro-3,1-benzoxazin-2-one, 3,1-Benzoxazine oder anderen Triarylmethanleukofarbstoffen.

Die Herstellung solcher druck- oder thermoempfindlicher Aufzeichnungsmaterialine geschieht in bekannter Weise.

Ein druckempfindliches Material besteht beispielsweise aus mindestens einem Paar von Blättern, die mindestens einen Farbbildner der Formeln I bis IV gelöst oder dis pergiert in einem nichtflüchtigen organischen Lösungsmittel und einen sauren Entwickler enthalten.

Solche Verfahren und Zubereitungen sind beispielsweise aus den US-Patentschriften 2 800 457, 2 800 458, 2 948 753, 3 096 189 und 3 193 404 und aus den deutschen Offenlegungsschriften 2 555 080 und 2 700 937 bekannt.

Um eine frühzeitige Aktivierung der in dem druckempfindlichen Aufzeichnungsmaterial vorhandenen Farbbildner zu verhindern, werden diese vorzugsweise in Mikrokapseln eingeschlossen, die sich in der Regel durch Druck zerbrechen lassen.

Als Kapselwandmaterialien eignen sich z.B. Gelatine/Gummi arabicum, Polyamide, Polyurethane, Polyharnstoffe, Polysulfonamide, Polyester, Polycarbonate, Polysulfonate, Polyacrylate und Phenol-, Melamin- oder Harnstoff-Formaldehyd-Kondensate, wie sie beispielsweise in M. Gutcho, Capsule Technology and Microencapsulation, Noyes Data Corporation 1972, G. Baster, Microencapsulation, Processes and Applications, Herausgeber J.E. Vandegaar und den deutschen Offenleungsschriften 2 237 545 und 2 119 933 beschrieben sind.

Bevorzugt werden Mikrokapseln verwendet, deren Hüllen aus Polyadditionsprodukten aus Polyisocyanaten und Polyaminen bestehen.

Isocyanate, Amine, Lösungsmittel und ein geeignetes Herstellungsverfahren für solche Mikrokapseln sind beispielsweise in DE-OS 3 203 059 beschrieben.

Ebenfalls bevorzugt werden Mikrokapseln verwendet, deren Hüllen aus Polyamiden oder Melamin-Formaldehyd-Kondensaten oder Gelatine/Gummi arabicum bestehen.

Als Entwickler sind Tone, sauer modifizierte Tone, Oxide oder saure Salze sowie monomere oder polymere Phenole oder Carbonsäuren zu nennen.

Besonders bevorzugte Farbentwickler sind Salze einer aromatischen Carbonsäure mit mindestens 10 Kohlenstoffatomen der Formel

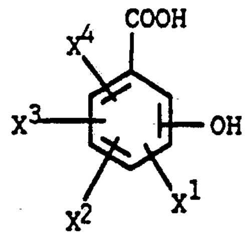

worin $X^1$, $X^2$, $X^3$ und $X^4$ Wasserstoff, Halogen, Hydrozyl, Alkyl, Cycloalkyl, Aryl, Aralkyl, Alkoxy oder Aryloxy bedeuten oder zwei benachbarte Reste $X^1$, $X^2$, $X^3$ und $X^4$ gemeinsam einen Ring bilden können, und wobei diese Reste die oben genannten, in der Farbstoffchemie üblichen nichtionischen Substituenten tragen können. Derartige Verbindungen sind z.B. in DE-OS 3 635 311 und 3 635 742 beschrieben

Die Tetraindolylheptamethinderivate der Formel I bis IV sind in den zur Mikroverkapselung üblichen nichtflüchtigen organischen Lösungsmitteln in der Regel ausreichend bis sehr gut löslich.

Thermoreaktive Aufzeichnungssysteme umfassen z.B. wärmeempfindliche Aufzeichnungs- und Kopiermaterialien und -papiere.

Ein solches Material ist beispielsweise in der DE-OS 2 555 080 beschrieben.

Als Entwickler eignen sich die gleichen Elektronenakzeptoren, wie sie in druckempfindlichen Papieren verwendet werden, vorzugsweise phenolische Verbindungen, die beispielsweise in der DE-PS 1 251 348 beschrieben sind, sowie Borsäure und organische, vorzugsweise aliphatische Dicarbonsäuren.

Ein weiteres geeignetes thermoreaktives Entwicklungssystem ist in DE-OS 3 337 296 beschrieben, bei dem sauer modifizierte Polymerisate, vorzugsweise des Acrylnitrils, als Entwickler wirken.

## Beispiel 1

29,5 g 1,1-Bis-(1-benzyl-2-phenyl-indol-3-yl)-ethen und 4,3 g 1,1,3,3=Tetramethoxypropan werden in einer Mischung aus 50 ml Acetanhydrid und 2,5 g Methansulfonsäure 1 h bei 80 °C verrührt. Die schwarzblaue lösung wird auf 200 ml Methanol ausgetragen und mit 8,9 g p-Toluolsulfinsäure-Natriumsalz versetzt. Nach Zusatz von 60 g wasserfreien Natriumcarbonat wird das grünlich beiges Pulver vom Schmelzpunkt 174 °C. I einer isomeren Form entspricht das Produkt der Formel:

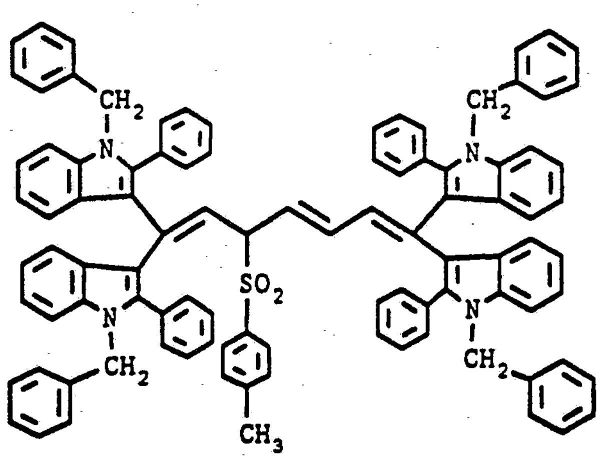

Eine Lösung in Eisessig zeigt eine schmutzig-blaue Farbe und $\lambda_{max}$ = 875 nm. Eine Lösung in Toluol entwickelt auf Säureton eine blasse graugrüne Färbung. Im Infrarot läßt sich eine Absorption von 750-950 nm messen..

Beispiel 2

25 g des Ethers der Formel

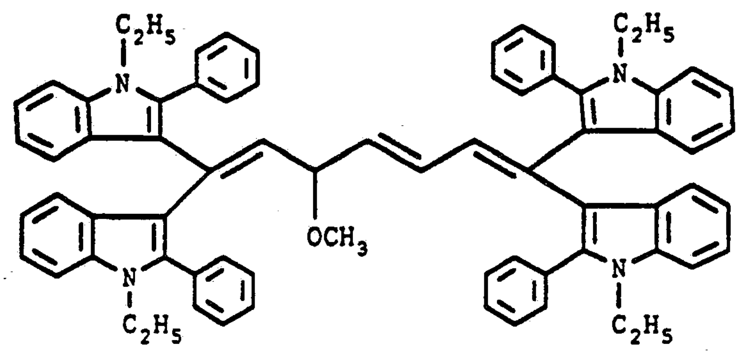

werden mit 3,5 g Methansumfonsäure in 100 ml Acetonitril gelöst. 7,5 g Phthalimid und 30 g wasserfreie Soda werden zugesetzt. 10 min wird bei 40 °C verrührt. Nach dem Abkühlen wird abgesaugt. Das Produkt wird in 100 ml Metahnol verrührt, abgesaugt und mit Methanol und Wasser gewaschen. Nach Trocknen erhält man 20,1 g (71,7 %) bräunlich beiges Pulver vom Schmelzpunkt 160-164 °C. In einer isomeren Form entspricht es der Formel:

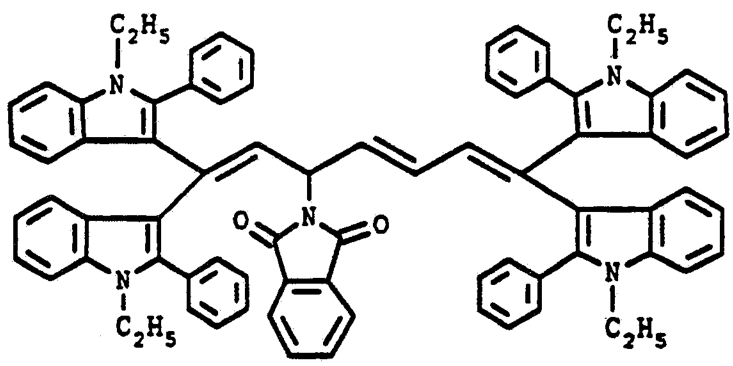

IR: 1 706 $cm^{-1}$.
$\lambda_{max}$ in Eisessig: 864 nm.
Auf Säureton: Grünlich grau, 750-950 nm.

Der als Edukt eingesetzte Ether wird wie folgt hergestellt:

23,3 g 1,1-Bis-(1-ethyl-2-phenyl-indol-3-yl)-ethen und 4,3 g 1,1,3,3-Tetramethoxypropan werden in einer Mischung aus 50 ml Acetanhydrid und 2,5 g Methansulfonsäure 1 h bei 90 °C verrührt. Die schwarzblaue Lösung, die den Farbstoff der Formel

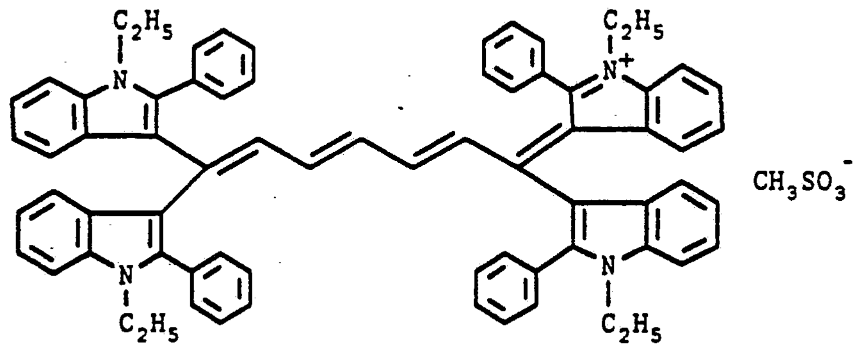

enthält, wird auf 200 ml Methanol ausgetragen, mit 70 ml 30%iger methanolischer Natriummethylatösung alkalsch gestellt und 2 h gekocht. Das beigebraune Produkt wird nach dem Abkühlen abgesaugt, mit Methanol und Wasser gewachen und getrocknet:
20,9 g (83,6 % der Theorie) bräunlich beiges Pulver vom Schmelzpunkt 176-178 °C.

Beispiel 3

25 g des Ethers der Formel

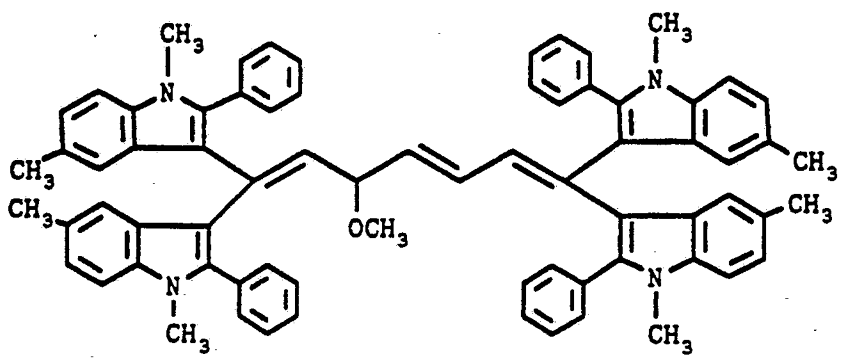

werden in 100 ml Acetonitril mit 3,4 g Methansulfonsäure zu einer blauen Farbstofflösung umgesetzt. 3,5 g 1,2,4-Triazol und 20 g wasserfreie Soda werden zugesetzt und 1/2 h bei 30-40 °C verrührt. Die Bräunlich beige Suspension wird kalt gerührt, abgesaugt und mit Methanol und Wasser gewaschen. Man erhält so 195 g (75 %) hellbeiges Pulver vom Schmelzpunkt 227-229 °C. In einer isomeren Form entspricht es der Formel:

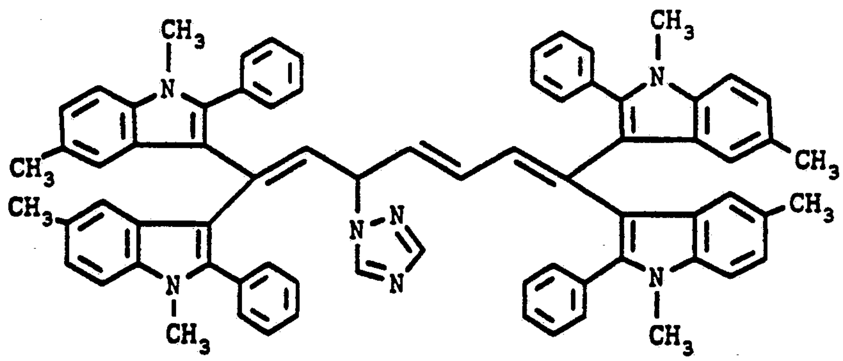

$\lambda_{max}$ in Eisessig: 863 nm.
Auf Säureton: Graublau, 750-950 nm.

Die Herstellung der hier und bei den folgenden Beispielen eingesetzten Ether erfolgt analog zu dem bei Beispiel 2 angegebenen Verfahren.

Beispiel 4

24,3 g des Ethers der Formel

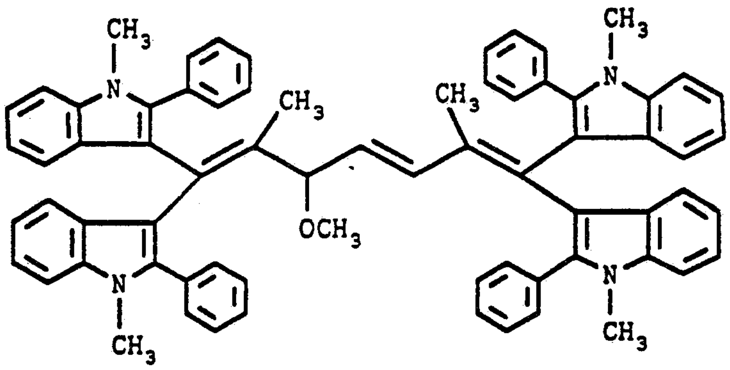

werden mit 2,65 g Methansulfonsäure in 130 ml Methanol 30 min bei 50° C verrührt. Dann werden 2,7 g Malodinitril und 8 g wasserfreie Soda zugesetzt. 7 h wird bei 50° C verrührt. Nach dem Abkühlen wird abgesaugt und mit Methanol und Wasser gewaschen. Das Produkt wird getrocknet und dann in 100 ml Ethanol aufgekocht. Nach dem Erkalten wird abgesaugt, mit Ethanol und Wasser gewaschen und getrocknet. Man erhält so 20,0 g (79,4 % der Theorie) bräunlich beiges Pulver vom Schmelzpunkt 194-200° C. In einer isomeren Form entspricht es der Formel:

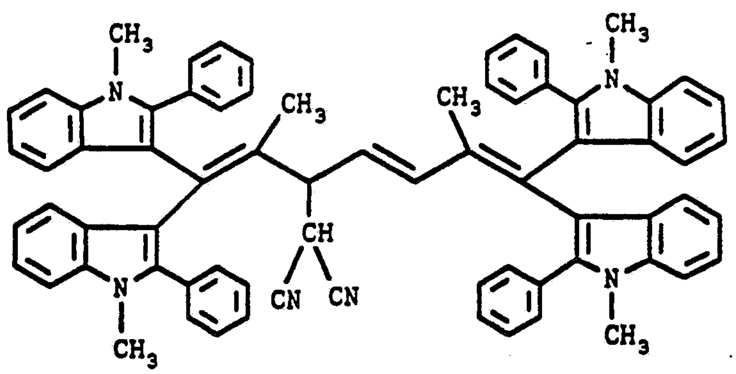

IR: 2 250 $cm^{-1}$.
$\lambda_{max}$ in Eisessig: 866 nm.
Auf Säureton: Grau, 750-950 nm.

Beispiel 5

5 g des Ethers der Formel

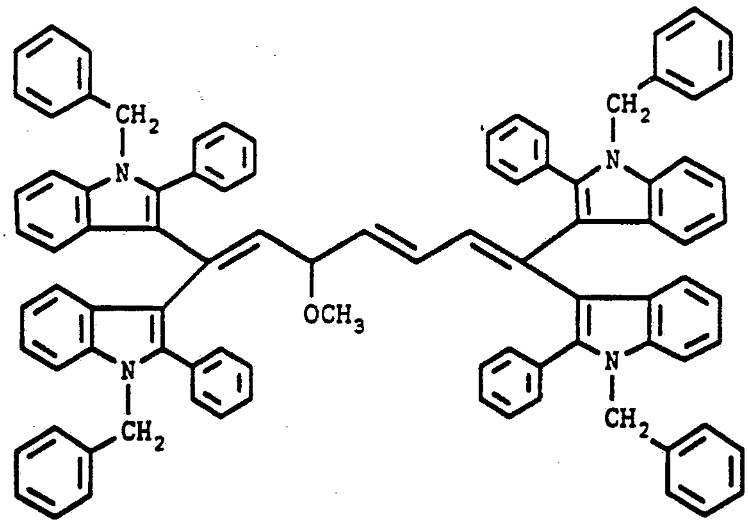

werden in 50 ml Methanol unter Zusatz von 1,5 ml Eisessig gelöst. 3 g Acetylaceton werden zugesetzt. Sofort bildet sich eine hellblaue suspension, die mit Kalium carbonat entfärbt wird. Es wird abgesaugt und mit Methanol und Wasser gewaschen. Man erhält so 5,3 g (99 % der Theorie) beiges Pulver vom Schmelzpunkt 152-155° C. In einer isomeren Form entspricht es der Formel:

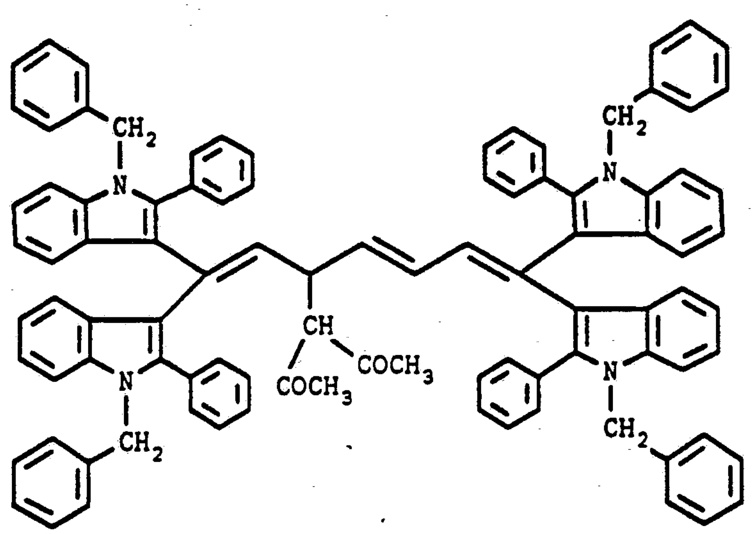

IR: 1 690 $cm^{-1}$.
$\lambda_{max}$ in Eisessig: 875 nm.
Auf Säureton: Grünlich grau, 750-950 nm.

Beispiel 6

5,4 g des Ethers der Formel

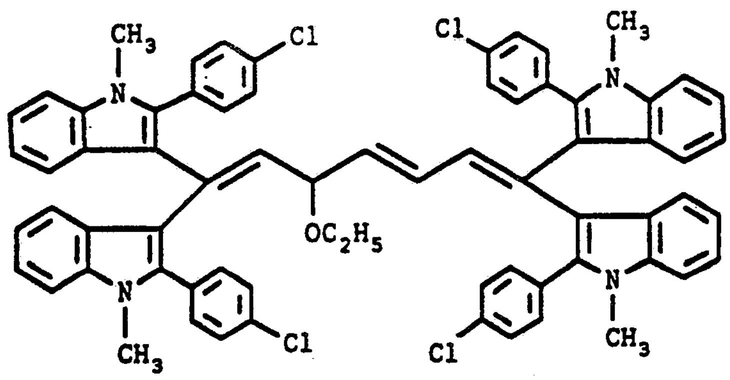

werden in 20 ml Acetonitril unter Zusatz von 2 g Eisessig in der Wärme mit blauer Farbe gelöst. 2,8 g Anilin und 8 g wasserfreie Soda werden zugesetzt und 3 h bei 40 °C verrührt. Allmählich fällt ein Niederschlag aus. Die Suspension wird kalt gerührt, abgesaugt und mit Acetonitril und Wasser gewaschen. Nach dem Trocknen erhält man 4,4 g (96 % der Theorie) beiges Pulver von Schmelzpunkt 170-172 °C.

In einer isomeren Form entspricht es der Formel:

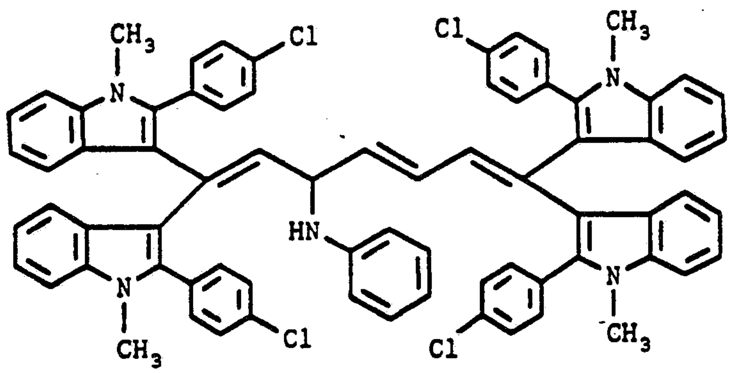

$\lambda_{max}$ in Eisessig: 866 nm.
Auf Säureton: Graugrün, 750-950 nm.

Beispiel 7

5 g des Ethers der Formel

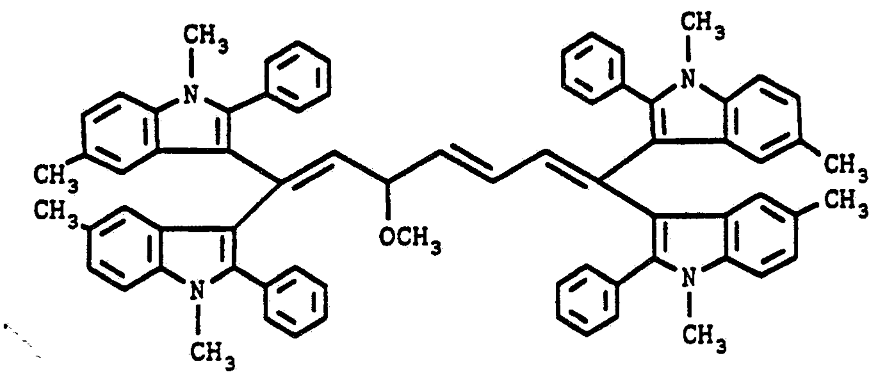

werden in 30 ml Methanol mit 0,7 g Methansulfonsäure gelöst. Bei Raumtemperatur werden 1,3 g 3-Metyl-1-phenyl-2-pyrazolin-5-on und 10 g wasserfreie Soda zugesetzt. Nach 1 h Rühren unter leichter Kühlung wird abgesaugt, mit Methanol und Wasser gewaschen und im Exsikkator getrocknet: 4,3 g (75,2 % der

Theorie) beiges Pulver vom Schmelzpunkt 160- 165 °C.
In einer isomeren Form entspricht es der Formel:

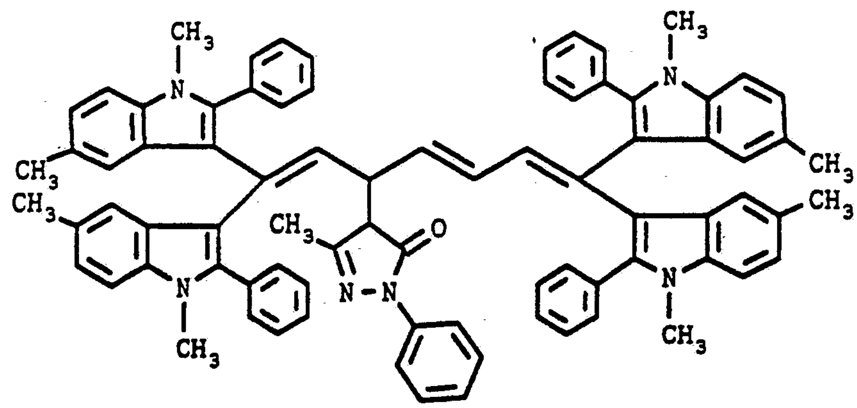

$\lambda_{max}$ in Eisessig: 863 nm.
Auf Säureton: Bläulich grau, 750-950 nm.

Beispiel 8

5,4 g des Ethers der Formel

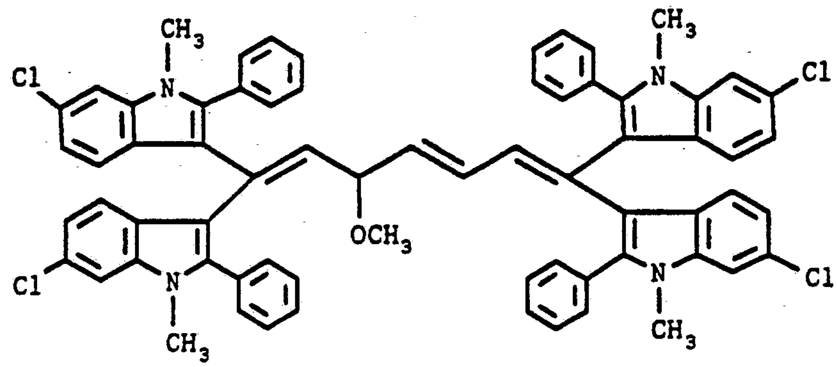

werden in 20 ml Acetonitril mit 0,73 g Meldrumsäure 4 h verrührt. Es wird auf 200 ml Wasser ausgetragen, abgesaugt, mit Wasser gewaschen und getrocknet: 4,5 g (75,4 % der Theorie) braunes Pulver vom Schmelzpunkt 200-205 °C (Zers.).
In einer isomeren Form entspricht es der Formel:

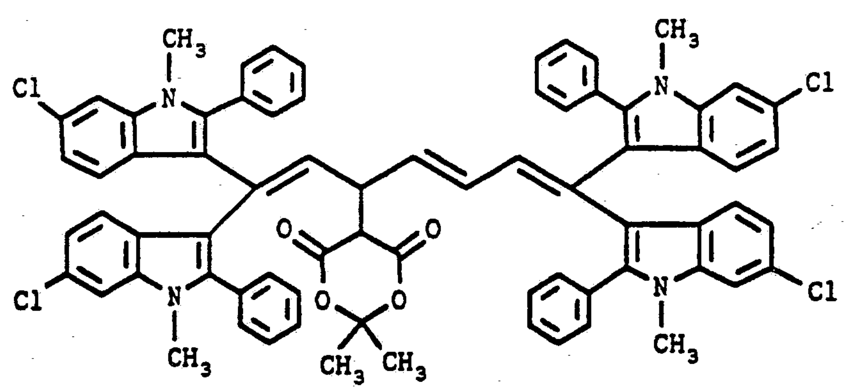

IR: 3 420, 1 741 $cm^{-1}$.
$\lambda_{max}$ in Eisessig: 865 nm.
Auf Säureton: Grünlich grau, 750-950 nm.

Beispiel 9

5,12 g des ethers der Formel

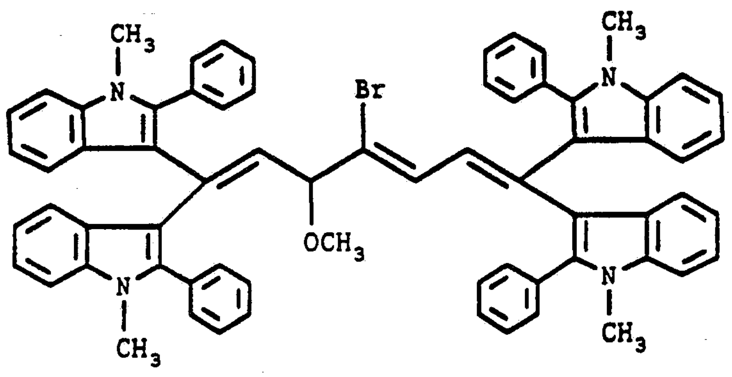

werden in 20 ml Acetonitril unter Zusatz von 0,67 g Methansulfonsäure bei 40 °C gelöst. 1,62 g 4-Nitrobenzylcyanid und 3,5 g wasserfreie Soda werden zugesetzt. Nach 20 min bei 40 °C wird abgekühlt. 50 ml Methanol werden zugesetzt. Es wird abgesaugt, mit Methanol und Wasser gewaschen und getrocknet: 4,5 g (78 % der Theorie) braunbeiges Pulver vom Schmelzpunkt 178-180 °C. In einer isomeren Form hat es die Formel:

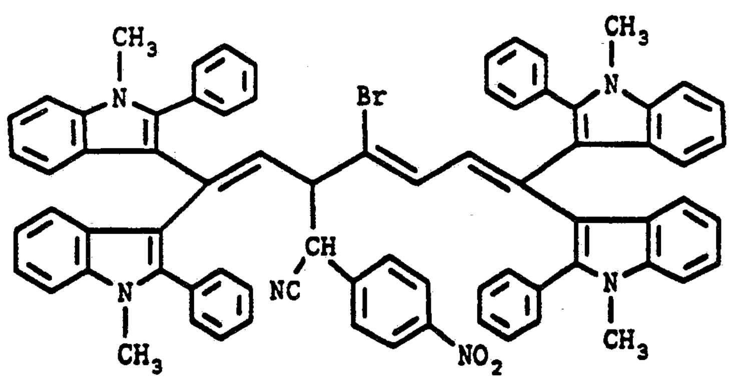

IR: 2 235, 1 520, 1 340 $cm^{-1}$.
$\lambda_{max}$ in Eisessig: 869 nm. Auf Säureton: Graugrün, 750-950 nm.

Beispiel 10

41 g des Ethers der Formel

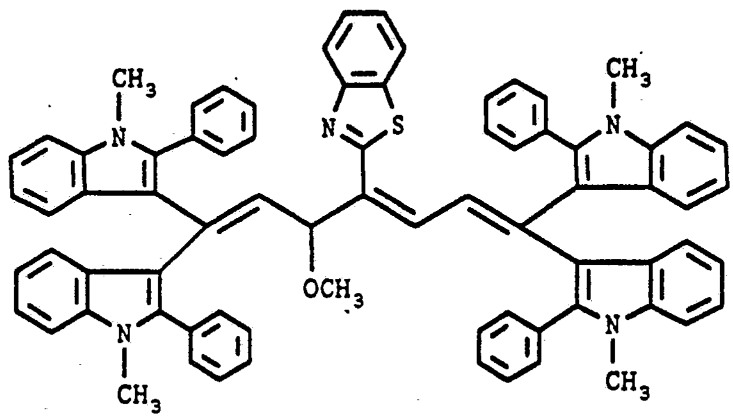

werden in 150 ml Acetonitril mit 5 g Methansulfonsäure in Lösung gebracht. 21 g Pyrrolidin tropfen dazu. Es entsteht eine gelbe Suspension, die abgesaugt, mit Acetonitril gewaschen und getrocknet wird: 34 g (74,3 % der Theorie) gelbes Pulver vom Schmelzpunkt 233-235 °C. In einer isomeren Form hat es die Formel:

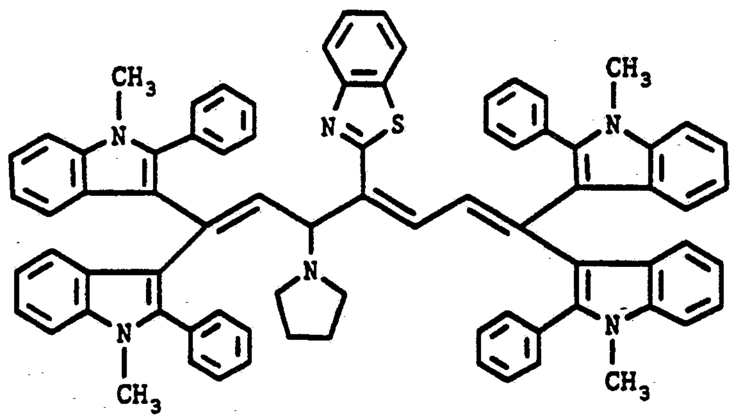

$\lambda_{max}$ in Eisessig: 859 nm.
Auf Säureton: Grünlich grau, 750-950 nm.

Analog Beispiel 1-10 lassen sich die folgenden Beispiele herstellen:

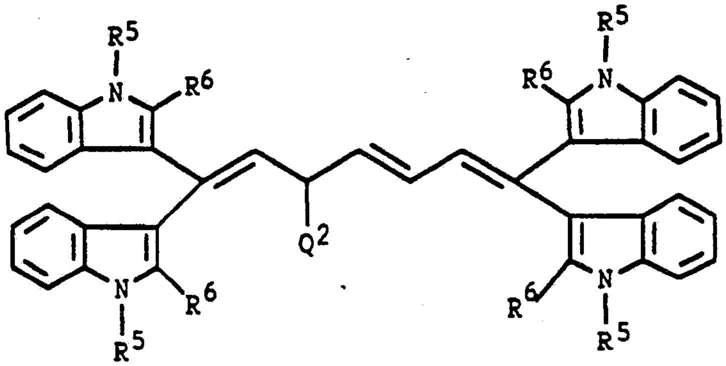

| Beisp. | $R^5$ | $R^6$ | $Q^2$ | $\lambda_{max}$ in Eisessig |
|---|---|---|---|---|
| 11 | $-CH_3$ | 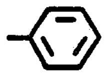 | $-SO_2C_8H_{17}$ | 863 nm |
| 12 | $n-C_4H_9$ | 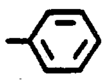 | 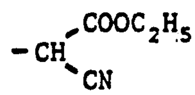 | 867 nm |
| 13 | $n-C_8H_{17}$ | 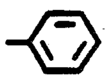 | 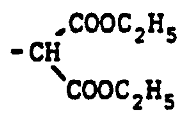 | 867 nm |
| 14 | $-CH_2$ 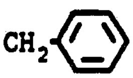 | 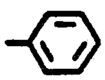 | -NHCO 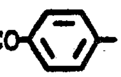 -Cl | 874 nm |
| 15 | $-CH_3$ | 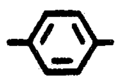 $OCH_3$ | $-SO_2$ 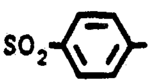 $CH_3$ | 870 nm |
| 16 | $-CH_3$ | 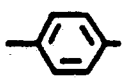 Cl | -NHCO 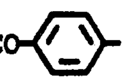 $NO_2$ | 865 nm |

| Beisp. | $R^5$ | $R^6$ | $Q^2$ | $\lambda_{max}$ in Eisessig |
|---|---|---|---|---|
| 17 | $-CH_3$ | 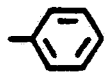 | 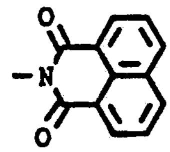 | 862 nm |
| 18 | $-CH_3$ | 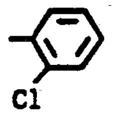 | 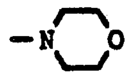 | 859 nm |
| 19 | $-CH(CH_3)_2$ | 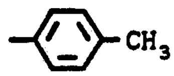 | 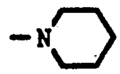 | 867 nm |
| 20 | $-CH_3$ | 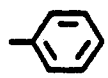 | 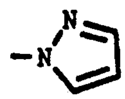 | 863 nm |
| 21 | $-CH_3$ | $-CH_3$ | 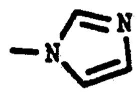 | 818 nm |
| 22 | 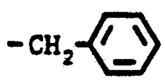 | $-CH_3$ | 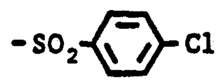 | 824 nm |
| 23 | $-CH_3$ | $n-C_4H_9$ | 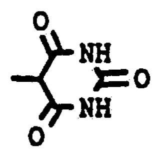 | 820 nm |
| 24 | $-CH_3$ | $n-C_8H_{17}$ | 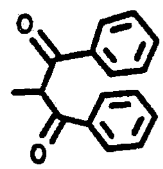 | 820 nm |
| 25 | $-CH_2CH_2CN$ | 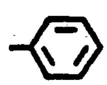 | $-NHSO_2CH_3$ | 852 nm |
| 26 | $-C_2H_4OCH_3$ | $n-C_6H_{13}$ | 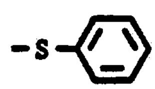 | 825 nm |

Beispiel 27

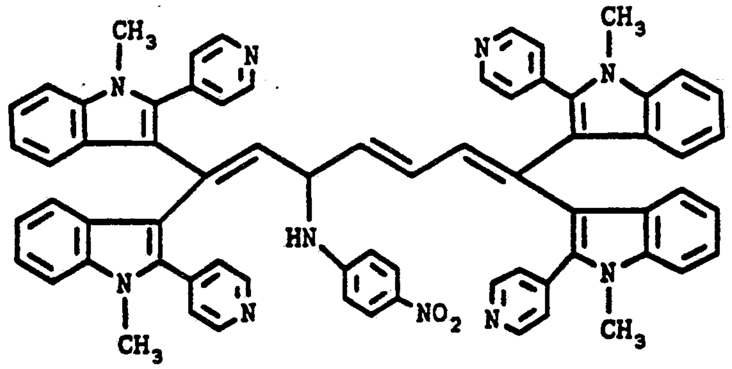

$\lambda_{max}$ in Eisessig: 857 nm.

Beispiel 28

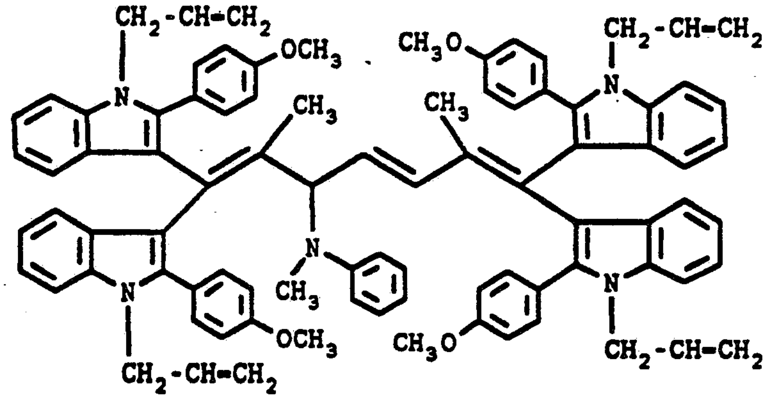

$\lambda_{max}$ in Eisessig: 872 nm.

Beispiel 29

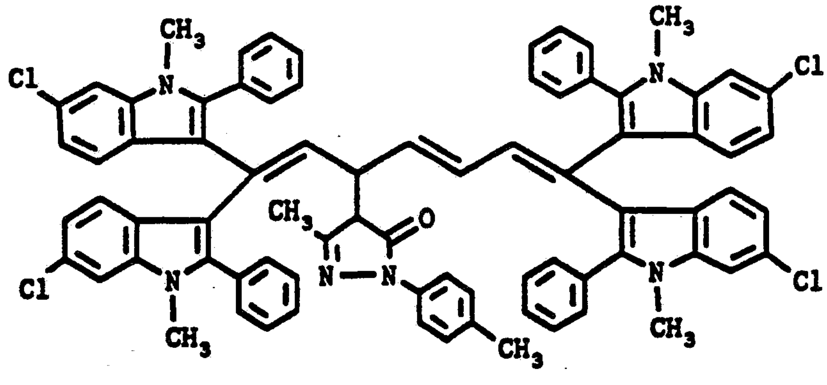

$\lambda_{max}$ in Eisessig: 865 nm.

Beispiel 30

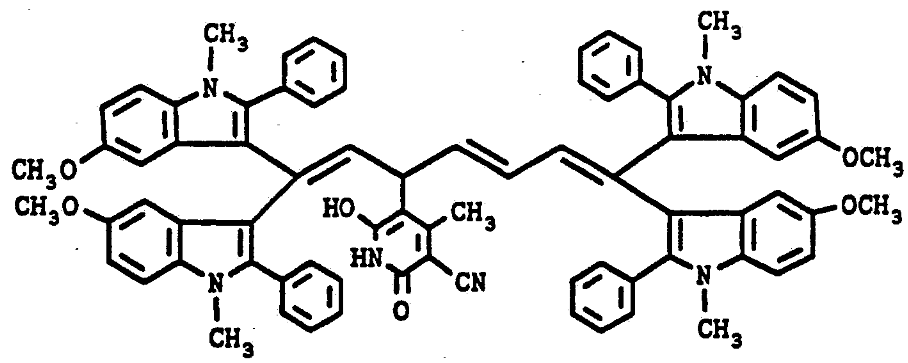

$\lambda_{max}$ in Eisessig: 870 nm.

Beispiel 31

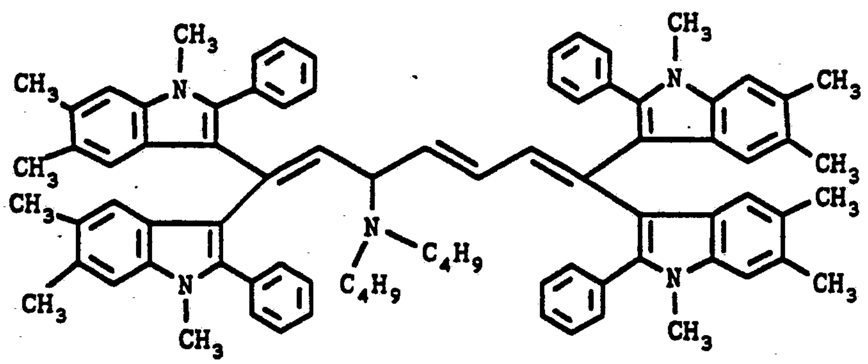

$\lambda_{max}$ in Eisessig: 865 nm.

Beispiel 32

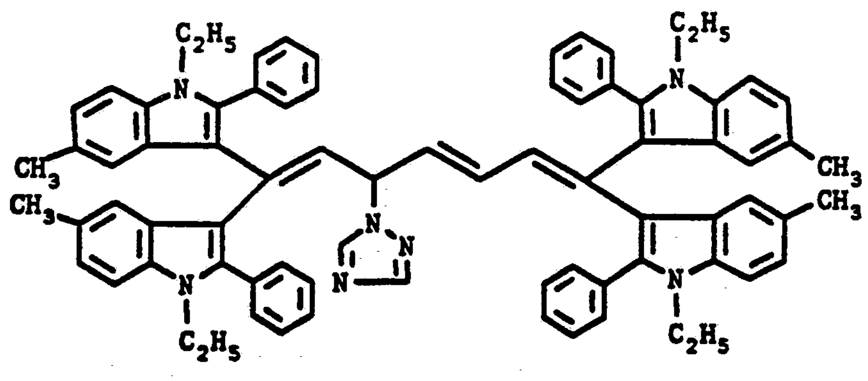

$\lambda_{max}$ in Eisessig: 864 nm.

Beispiel 33

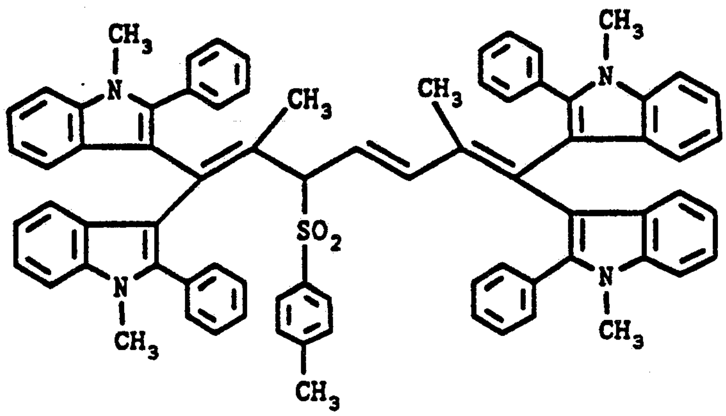

$\lambda_{max}$ in Eisessig: 862 nm.

Beispiel 34

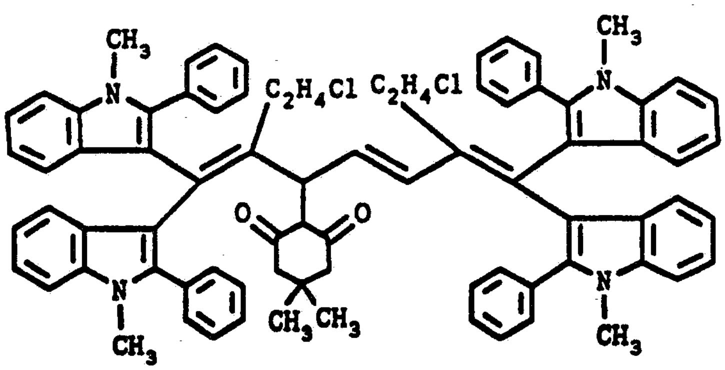

$\lambda_{max}$ in Eisessig: 867 nm.

Beispiel 35

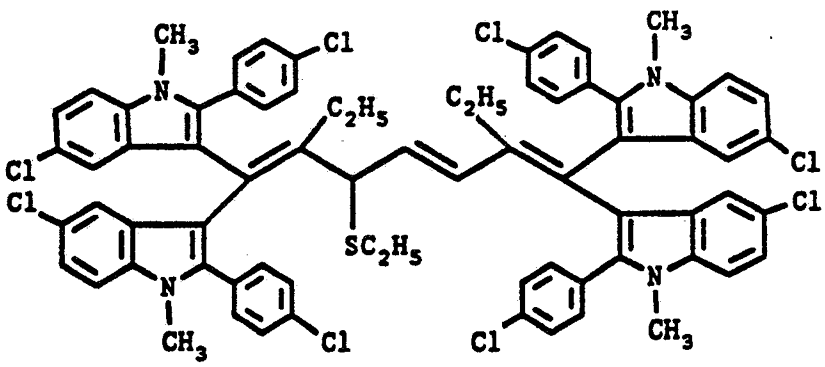

$\lambda_{max}$ in Eisessig: 863 nm.

Beispiel 36

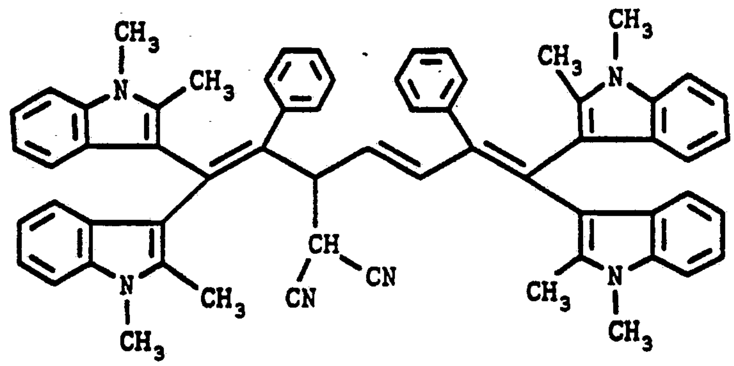

$\lambda_{max}$ in Eisessig: 800 nm.

Beispiel 37

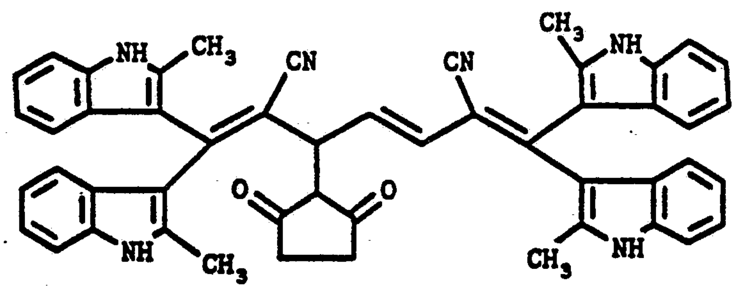

$\lambda_{max}$ in Eisessig: 791 nm.

Beispiel 38

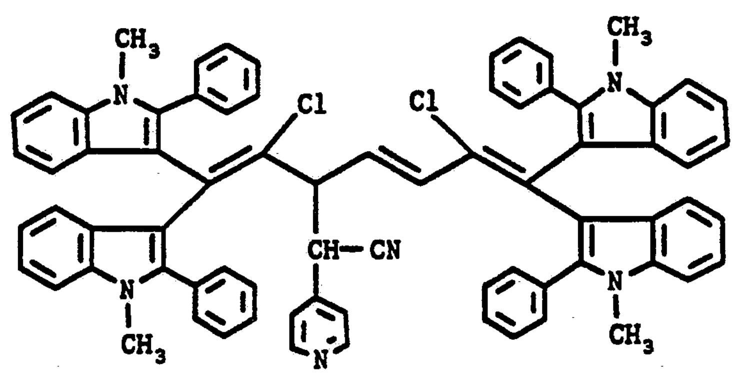

$\lambda_{max}$ in Eisessig: 900 nm.

Beispiel 39

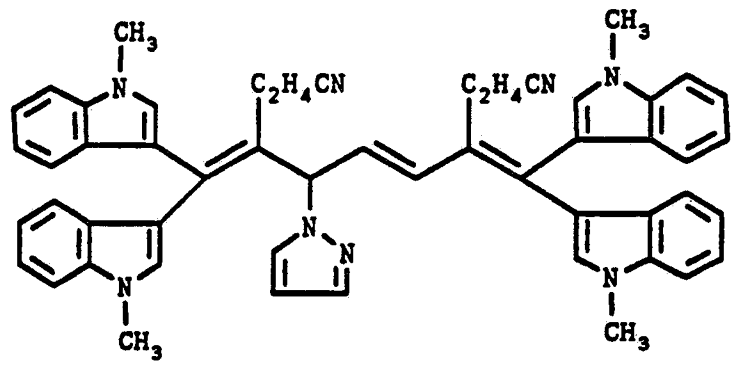

$\lambda_{max}$ in Eisessig: 815 nm.

Beispiel 40

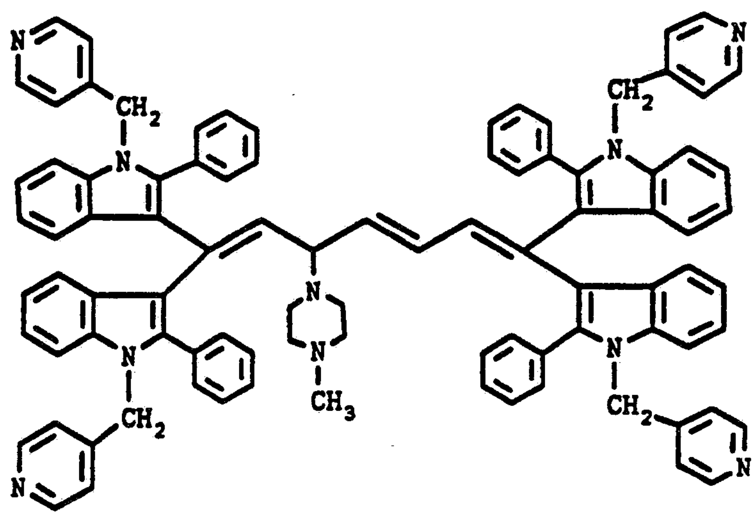

$\lambda_{max}$ in Eisessig: 870 nm.

Beispiel 41

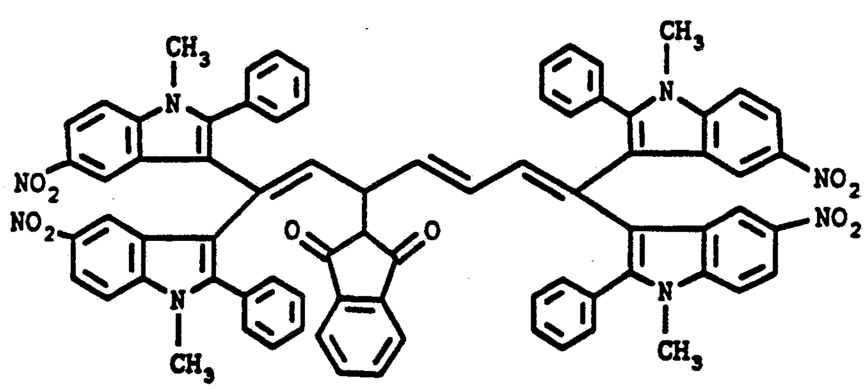

$\lambda_{max}$ in Eisessig: 858 nm.

Beispiel 42

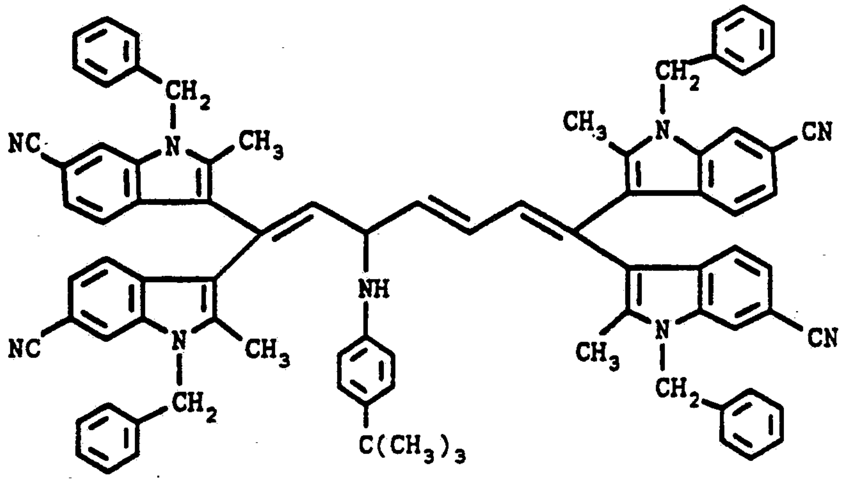

$\lambda_{max}$ in Eisessig: 830 nm.

Beispiel 43

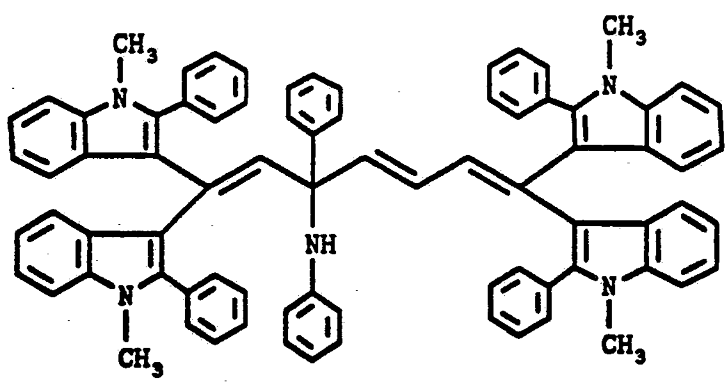

$\lambda_{max}$ in Eisessig: 897 nm.

Beispiel 44

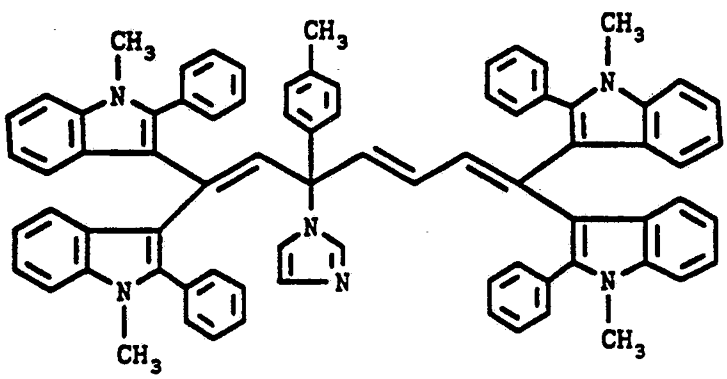

$\lambda_{max}$ in Eisessig: 895 nm.

Beispiel 45

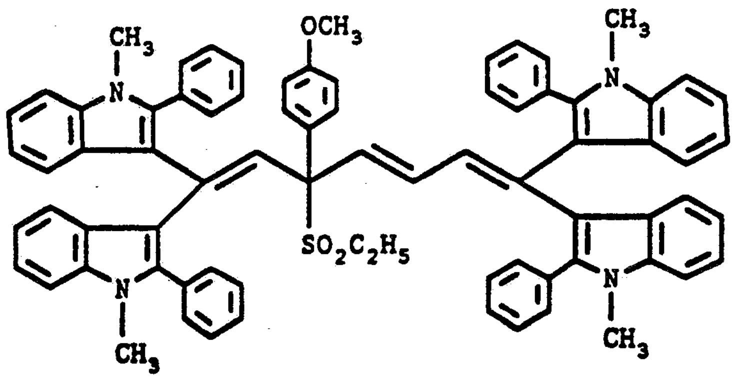

$\lambda_{max}$ in Eisessig: 885 nm.

Beispiel 46

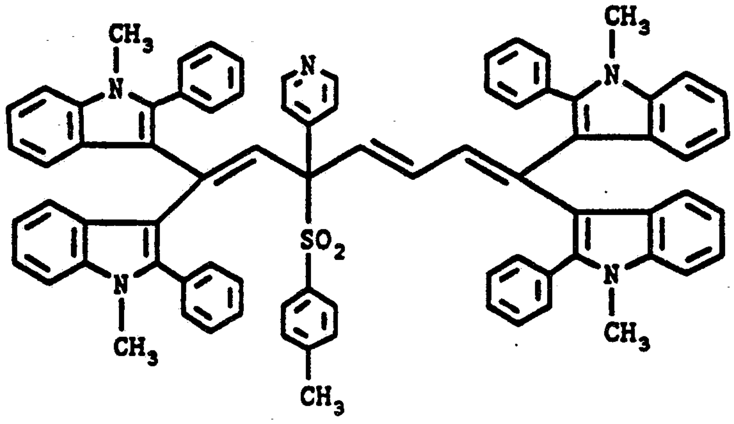

$\lambda_{max}$ in Eisessig: 905 nm.

Beispiel 47

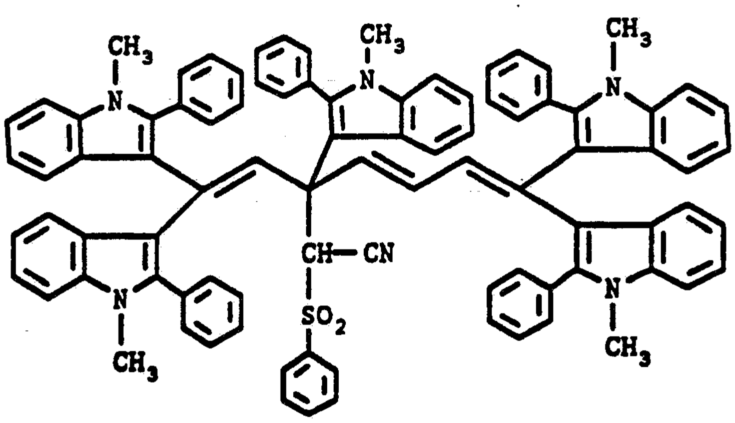

$\lambda_{max}$ in Eisessig: 876 nm.

Beispiel 48

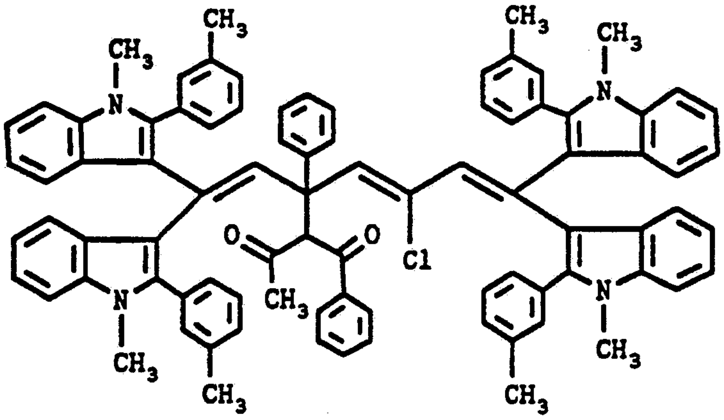

$\lambda_{max}$ in Eisessig: 900 nm.

Beispiel 49

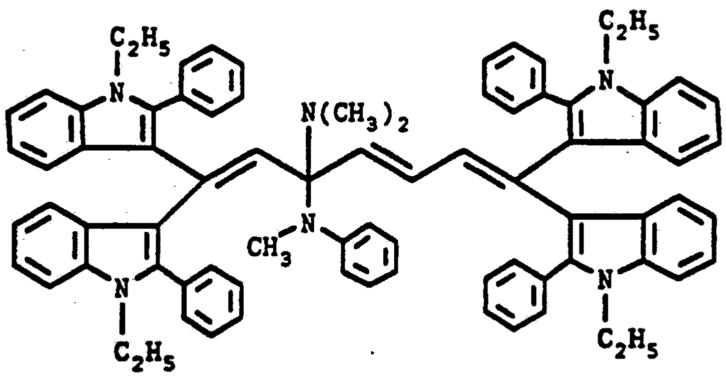

$\lambda_{max}$ in Eisessig: 770 nm.

Beispiel 50

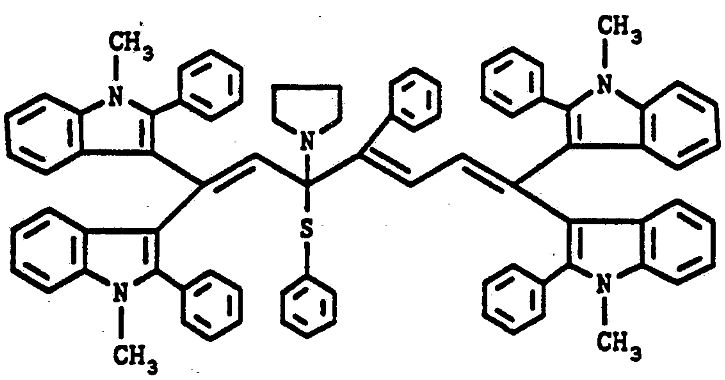

max in Eisessig: 802 nm.

Beispiel 51

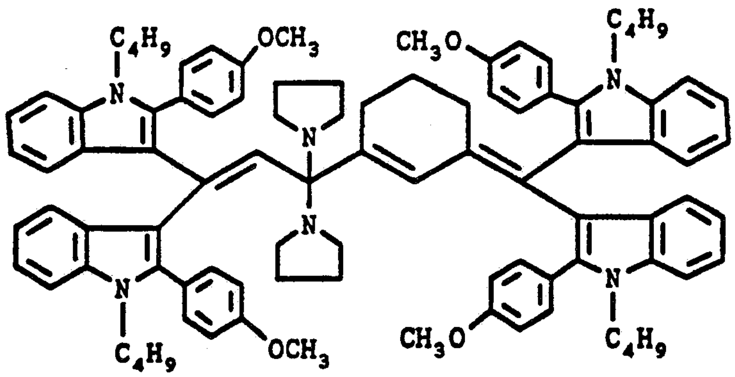

$\lambda_{max}$ in Eisessig: 765 nm.

Beispiel 52

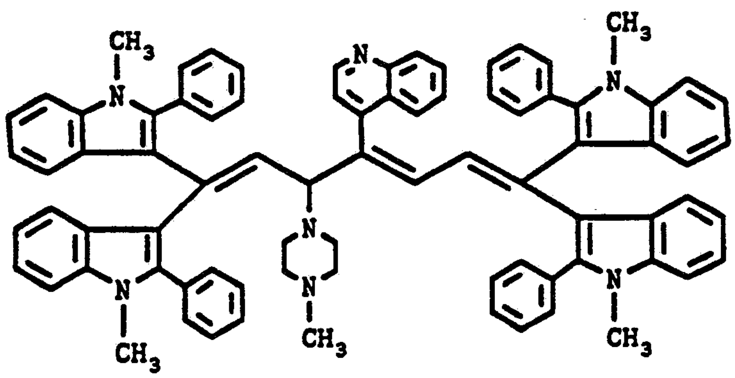

$\lambda_{max}$ in Eisessig: 865 nm.

Beispiel 53

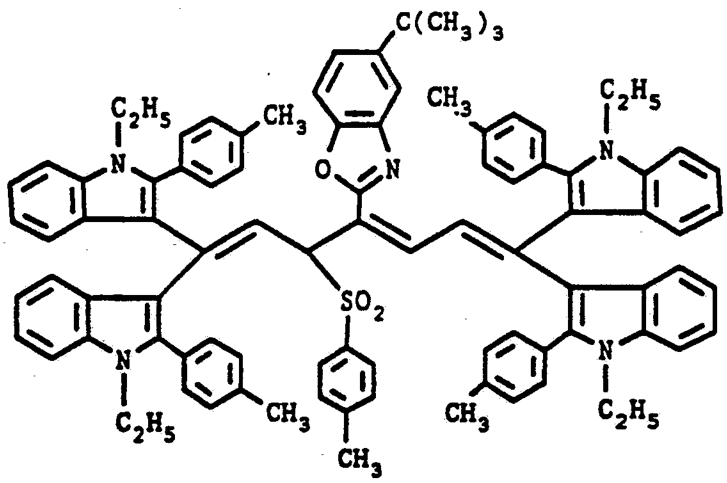

$\lambda_{max}$ in Eisessig: 860 nm.

Beispiel 54

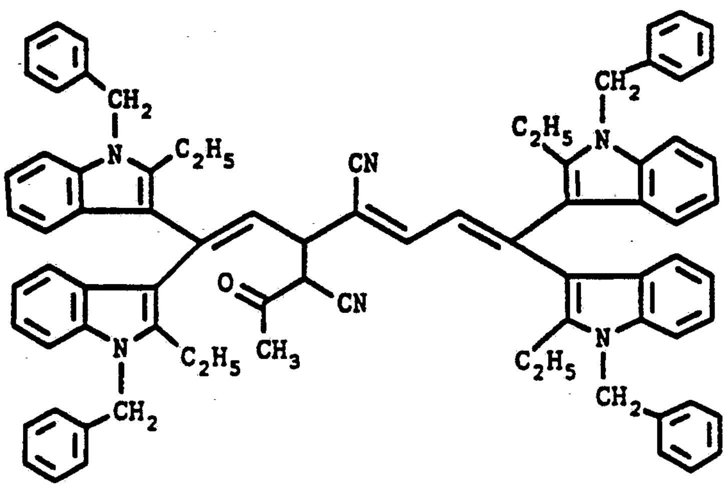

$\lambda_{max}$ in Eisessig: 800 nm.

Beispiel 55

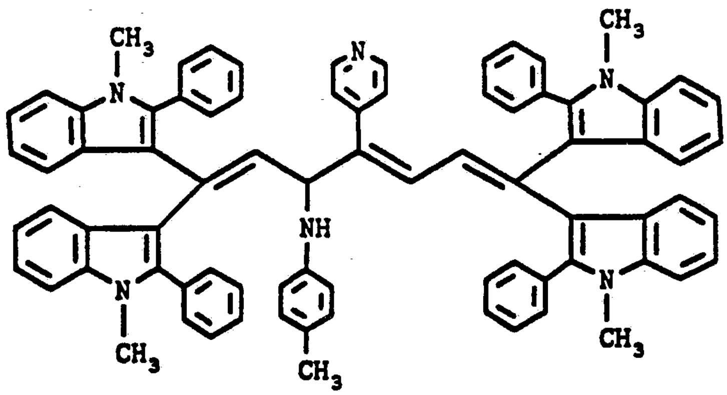

$\lambda_{max}$ in Eisessig: 850 nm.

Beispiel 56

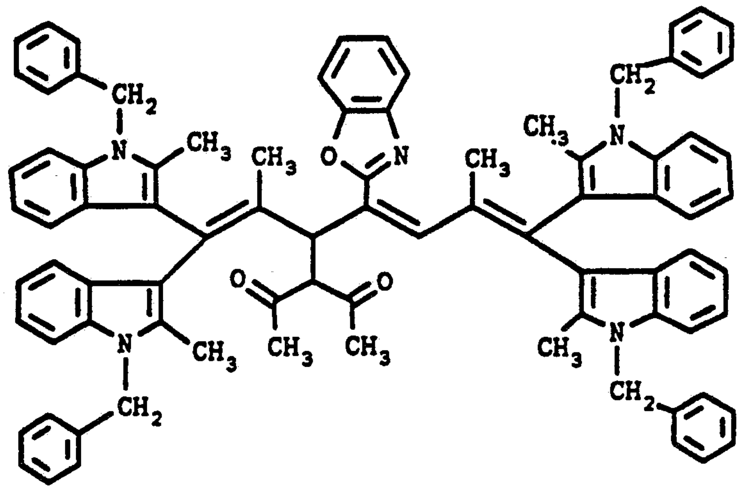

$\lambda_{max}$ in Eisessig: 815 nm.

Beispiel 57

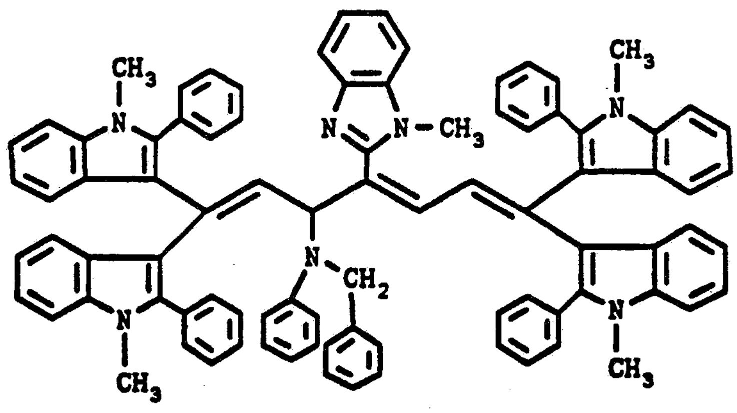

$\lambda_{max}$ in Eisessig: 858 nm.

Beispiel 58

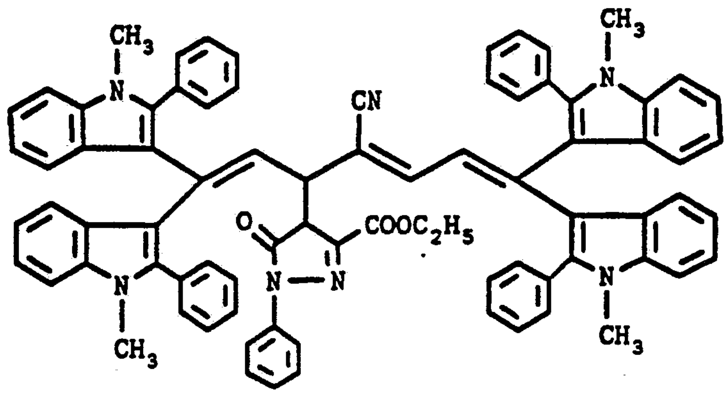

$\lambda_{max}$ in Eisessig: 800 nm.

Beispiel 59

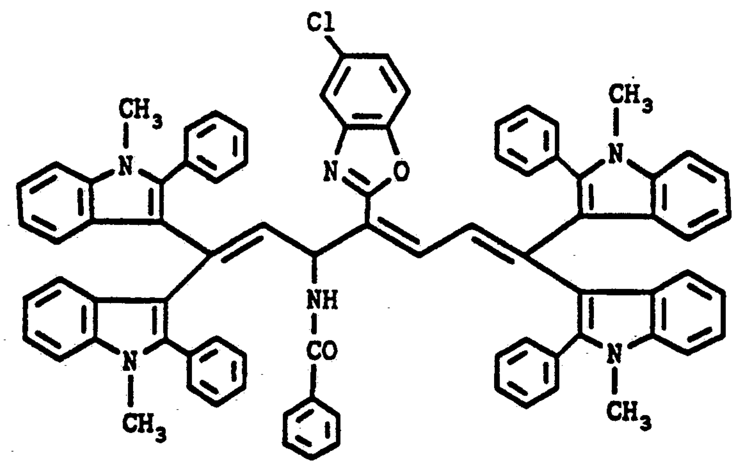

$\lambda_{max}$ in Eisessig: 859 nm.

Beispiel 60

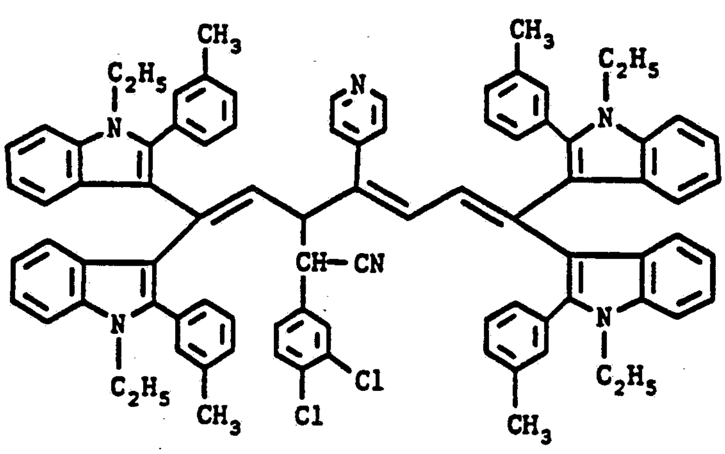

$\lambda_{max}$ in Eisessig: 851.

Beispiel 61

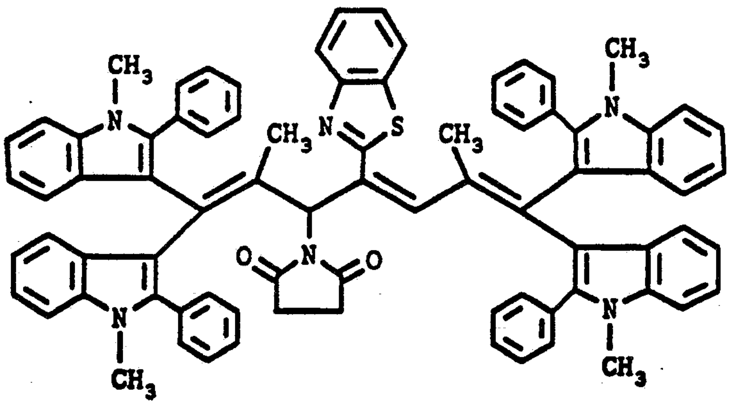

$\lambda_{max}$ in Eisessig: 859 nm.

Beispiel 62

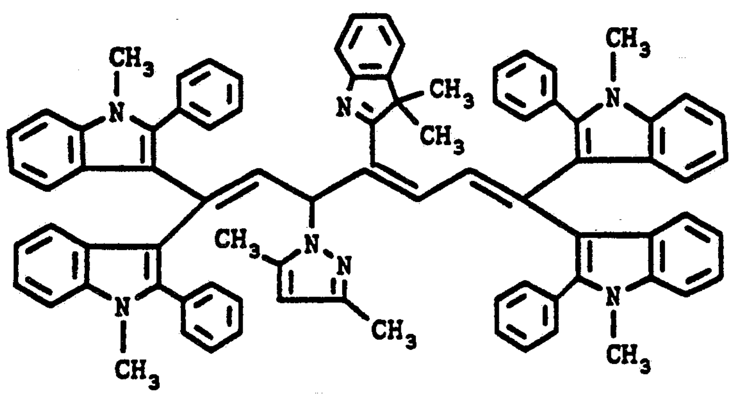

$\lambda_{max}$ in Eisessig: 857 nm.

Beispiel 63

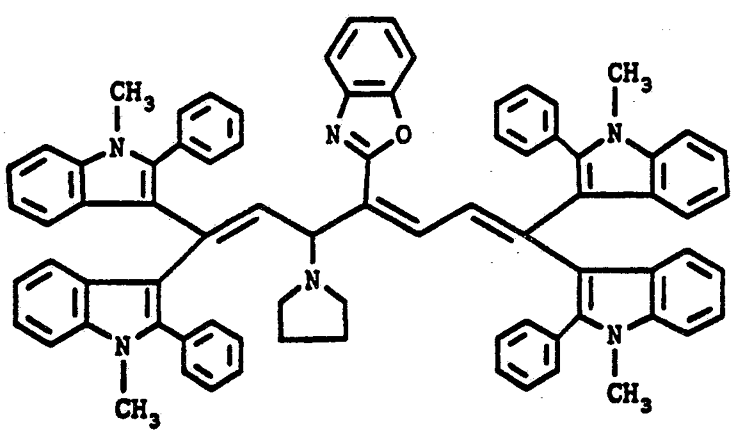

$\lambda_{max}$ in Eisessig: 858

Beispiel 64

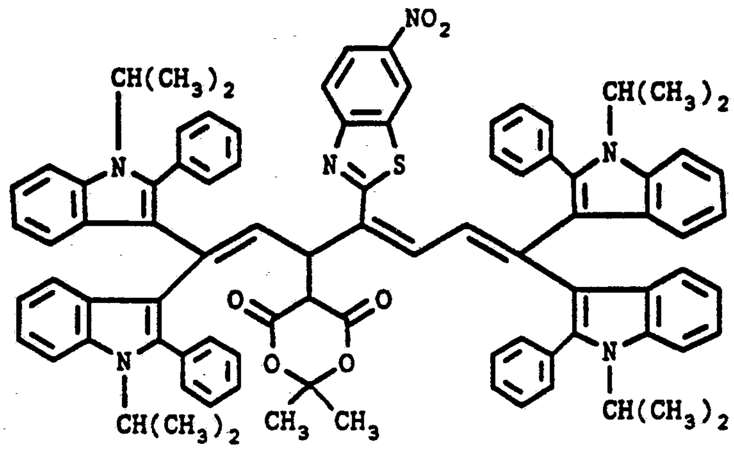

$\lambda_{max}$ in Eisessig: 859

Beispiel 65

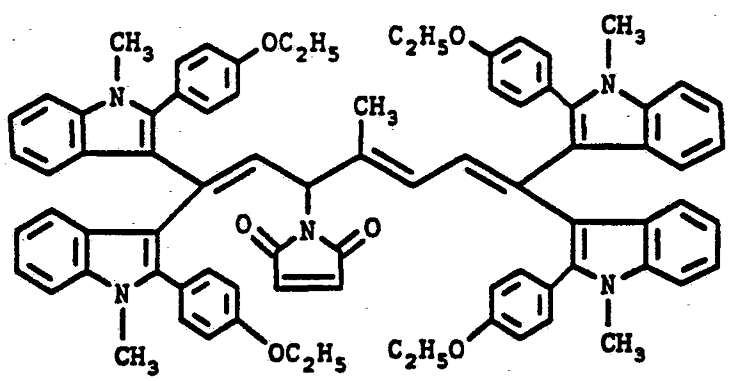

$\lambda_{max}$ in Eisessig:857 nm.

Beispiel 66

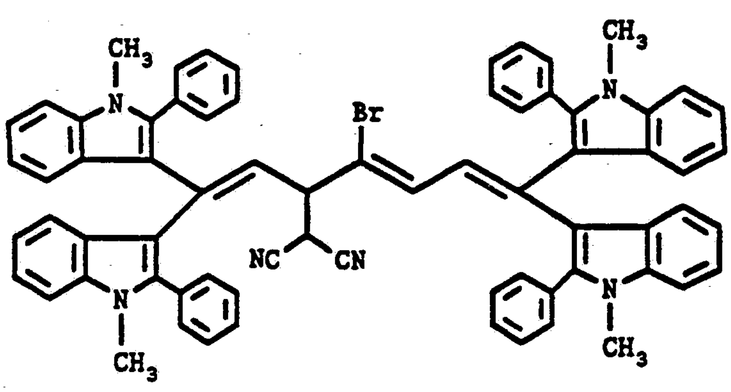

$\lambda_{max}$ in Eisessig: 869 nm.

Beispiel 67

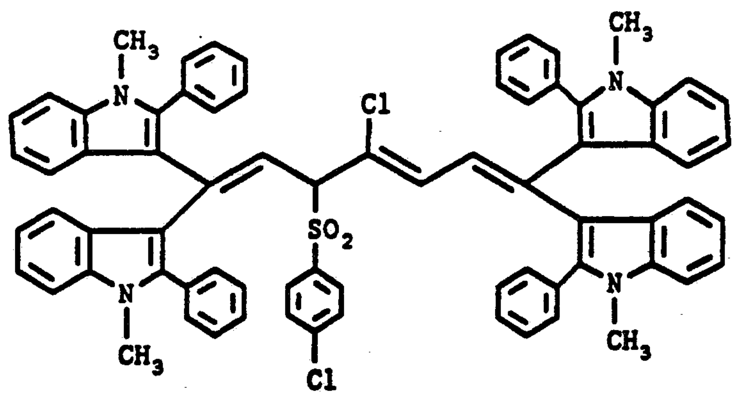

$\lambda_{max}$ in Eisessig: 874 nm.

Beispiel 68

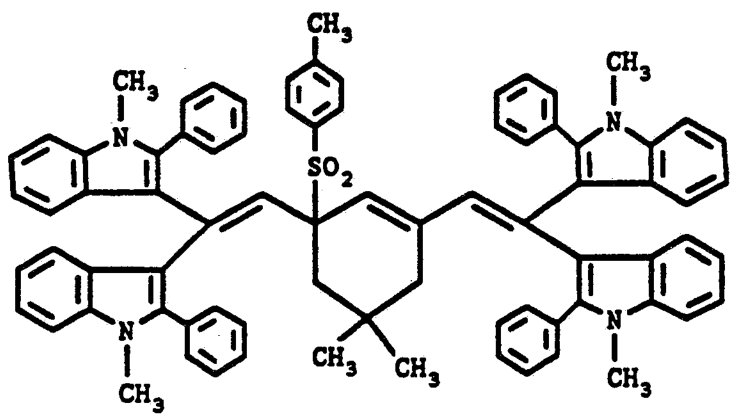

$\lambda_{max}$ in Eisessig: 797 nm.

Beispiel 69

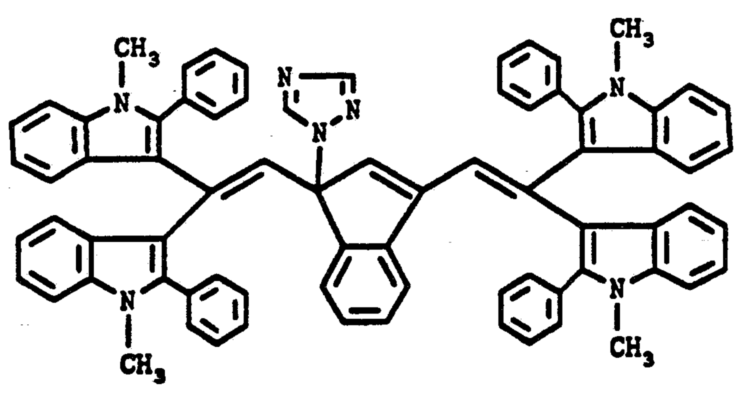

$\lambda_{max}$ in Eisessig: 794 nm.

Beispiel 70

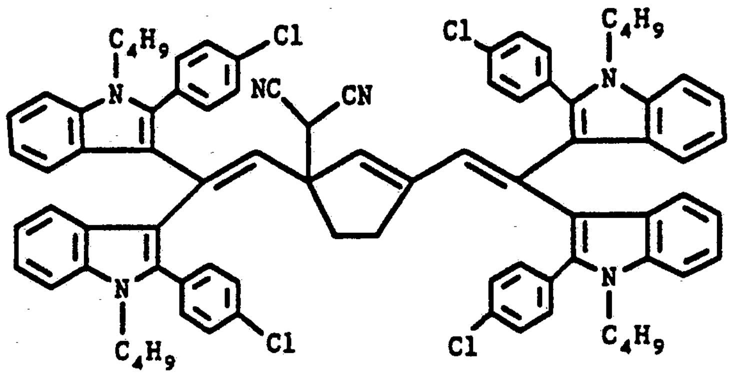

$\lambda_{max}$ in Eisessig: 803 nm.

Beispiel 71

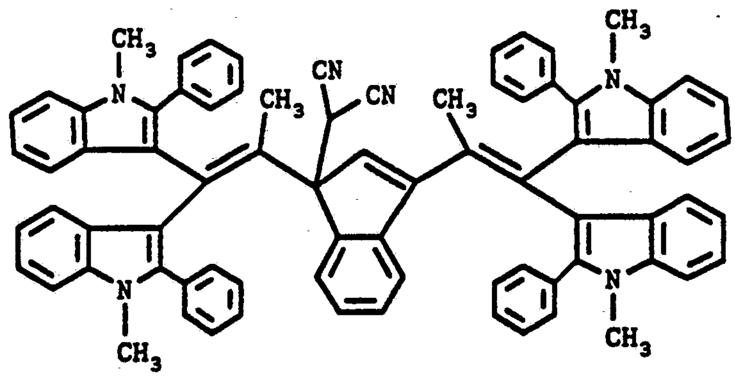

$\lambda_{max}$ in Eisessig: 795 nm.

Beispiel 72

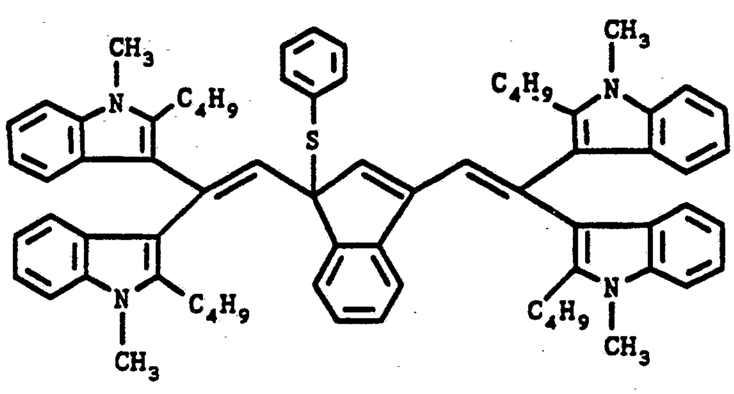

$\lambda_{max}$ in Eisessig: 760 nm.

Beispiel 73

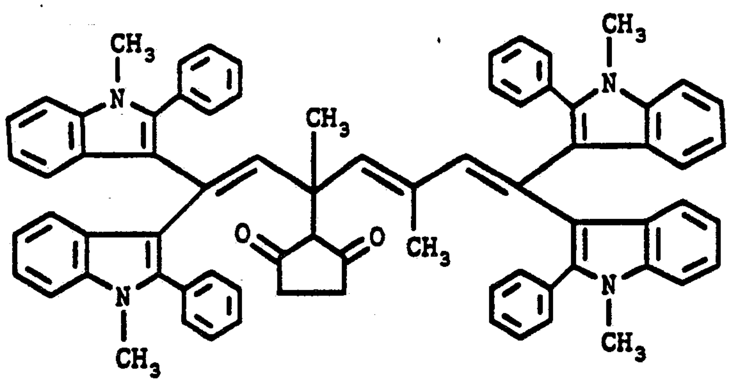

$\lambda_{max}$ in Eisessig: 798 nm.

Beispiel 74

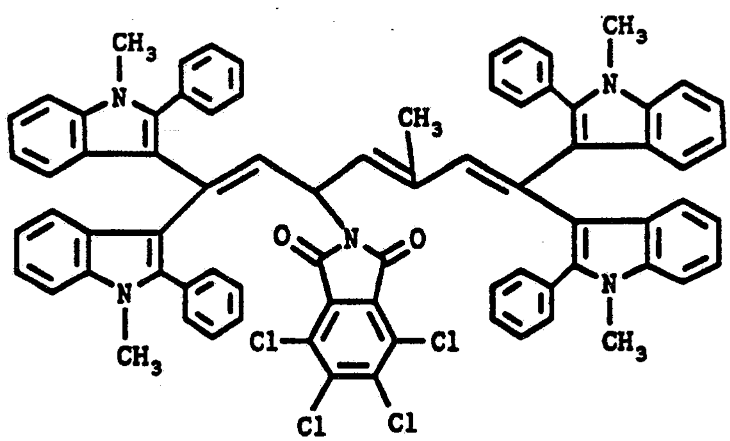

$\lambda_{max}$ in Eisessig: 880 nm.

Beispiel 75

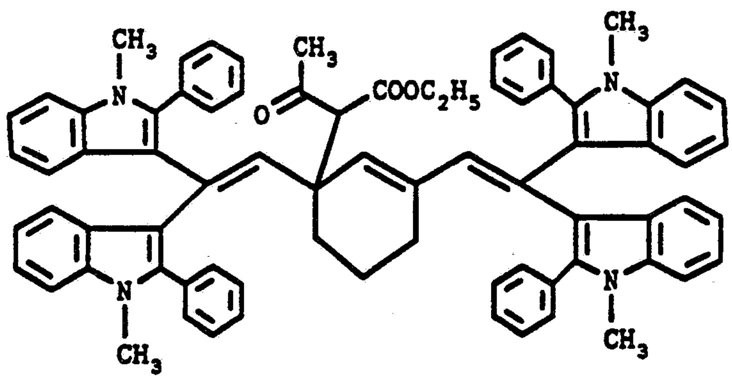

$\lambda_{max}$ in Eisessig: 796 nm.

Beispiel 76

11,3 g 1,1-Bis-(1-methyl-2-phenyl-indol-3-yl)-propen, 12,5 g 1,1-Bis-(1-methyl-2-(4-methoxy-phenyl)-indol-3-yl)-ethen und 4,3 g 1,1,3,3-Tetramethoxy-propan werden wie in Beispiel 1 umgesetzt. Man erhält 26,2 g bräunlich beiges Pulver, das aus einer Mischung (ca. 3:3:1) der Produkte der Beispiele 15, 33 und der Verbindung der Formel (in einer isomeren Form)

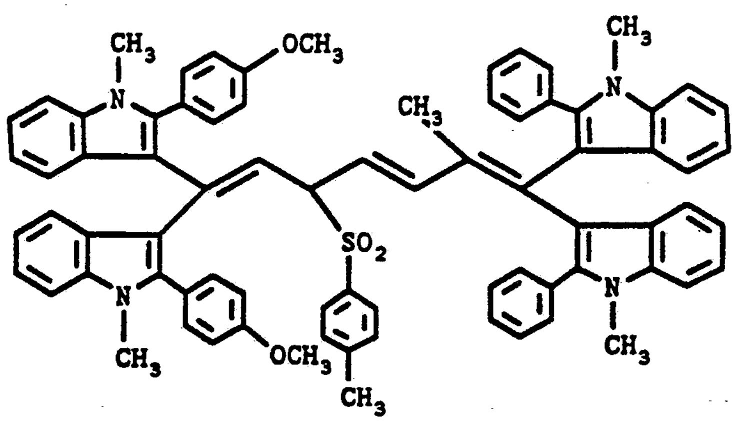

besteht.
$\lambda_{max}$ in Eisessig: 866 nm.
Auf Säureton: Grünlich grau, 750-950 nm.

Beispiel 77

3 g des Tetraindolylheptamethinderivates des Beispiels 1 werden in einem Gemisch aus 40 g Dodecylbenzol und 60 g Chlorparaffin mit 45 % Cl-Gehalt (Marlican der Fa. Hüls) gelöst. 223 g einer solchen Lösung werden mit 39,5 g Oxadiazintrion von Desmodur H (NCO-Gehalt: 20,5 %) vermischt. Anschließend erfolgt die Vermischung mit 320 g 0,5%iger Polyvinylalkohollösung und die Emulgierung im Schergefälle eines Rotor/Stator-Emulgiergerätes. Die Vernetzung erfolgt mit 76 g 9,0%iger Diethylentriaminlösung. Die Nachbehandlung erfolgt durch Erwärmen der Dispersion auf 60° C und dreistündiges Rühren bei 60° C. Es wird dabei eine 40 % Kapseln enthaltende Dispersion der Kapselgröße 7,3 µm erhalten.

250 ml dieser Dispersion werden vorgelegt und unter intensivem Rühren 40 g Cellulosefeinschliff (Arbocell BE 600/30 der Fa. Rettenmeier & Söhne) langsam eingestreut.

Nach mindestens 30-minütigem intensivem Rühren erfolgt die Zugabe von 40 ml 50%iger SBR-Latex (Baystal D 1600 der Fa. Bayer AG). Die resultierende 48,5%ige Streichfarbe wird mit Wasser auf 30 % Feststoffgehalt verdünnt und mit einer Luftbürste auf die Rückseite eines handelsüblichen Basis-Papiers gestrichen. Der Auftrag beträg nach dem Trocknen 5 g/m$^2$.

Das so bestrichene Papier wird mit der beschichteten Seite auf die mit Entwicklersubstanz beschichtete Seite eines handelsüblichen kohlefreien Durchschreibepapiers gelegt. Bei Ausführung eines Schreibdruckes auf das mit Kapseln beschichtete Papier ergibt sich auf dem Durchschreibepapier eine graublaue Durchschrift, die im nahen Infrarotbereich von 750-950 nm eine intensive Absorption besitzt.

Analog lassen sich auch die anderen Beispiele verwenden.

Beispiel 78

Eine Lösung aus 2 g des Tetraindolylheptamethinderivates des Beispiels 4 und 3 g eines Benzoxazins der Formel

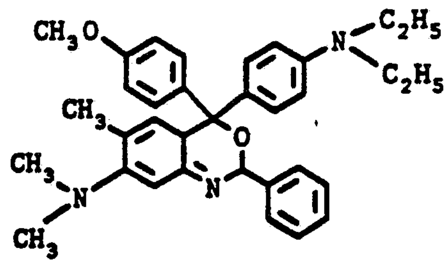

wie es in EP 187 329 beschrieben ist, in 80 g Diisopropylnaphthalin und 17 g Kerosin wird auf an sich bekannte Weise mit Gelatine und Gummi arabicum durch Koazervation mitroverkapselt, mit Stärkelösung vermischt und auf ein Blatt Papier gestrichen. Dieses Blatt wird mit der beschichteten Seite auf die mit Entwicklersubstanz beschichtete Seite eines handelsüblichen kohlefreien Durchscreibepapiers gelegt. Bei Ausübung eines Schreibdrucks auf das mit Kapseln beschichtete Papier ergibt sich auf dem Durchschreibepapier eine intensive schwarze Durchschrift, die im nahen Infrarot von 750-950 nm ebenfalls eine intensive Absorption besitzt.

Analog lassen sich auch die anderen Beispiele verwenden.

Beispiel 79

In einer Kugelmühle werden 32 g 4,4′-Isopropyliden-diphenol (Bisphenol A), 3,8 g Distearylamid des Ethylendiamins, 89 g Kaolin, 20 g eines zu 88 % hydrolysierten Polyvinylalkohols und 55 ml Wasser gemahlen, bis die Teilchengröße ca. 5 µm beträgt. In einer 2. Kugelmühle werden 6 g des Tetraindolylheptamethinderivates des Beispiels 2, 3 g eines zu 88 % hydrolysierten Polyvinylalkohols und 60 ml Wasser zu einer Teilchengröße von ca. 3 µm gemahlen. Die beiden Dispersionen werden zusammengegeben und mit einem Trockenauftragsgewicht von 5,5 g/m$^2$ auf Papier gestrichen. Durch Berühren des Papiers mit einem erhitzten Kugelschreiber wird eine grünlich graue Aufzeichnung entwickelt, die im nahen Infrarot von 750-950 nm eine intensive Absorption zeigt.

Analog lassen sich auch die anderen Beispiele verwenden.

**Ansprüche**

1. Tetraindolylheptamethinderivate der isomeren Formeln

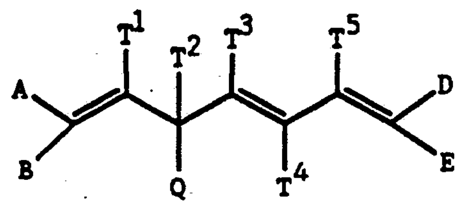

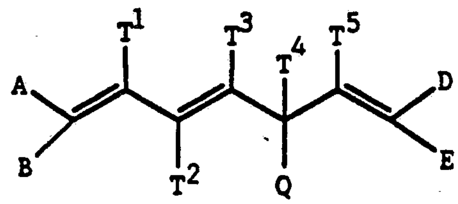

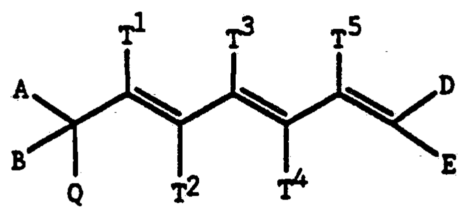

und

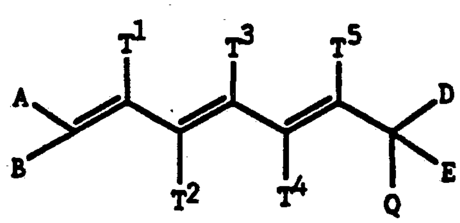

worin A, B, D und E

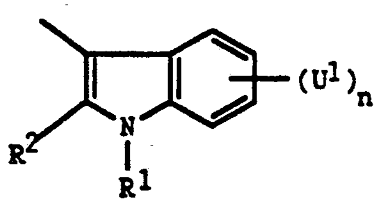

bedeuten und untereinander gleich ober verschieden sein können,
Q $NV^1V^2$, SW, $SO_2X$ oder $CLY^1Y^2$,
$R^1$ Wasserstoff, Alkyl, Alkenyl, Cycloalkyl, Aralkyl oder ein über Alkyl gebundener heterocyclischer Rest,
$R^2$ Wasserstoff, Alkyl, Alkenyl, Cycloalkyl, Aralkyl, Aryl oder ein gegebenenfalls über Alkyl gebundener heterocyclischer Rest,
$T^1$ bis $T^5$ Wasserstoff, Alkyl, Alkenyl, Cycloalkyl, Aralkyl, Halogen, Alkoxy, Dialkylamino, Cyano, Hydroxycarbonyl, Alkoxycarbonyl, Aryl, ein ggf. über Alkyl gebundener heterocyclischer Rest oder jeweils zwei der Reste $T^1$ bis $T^5$ die fehlenden Glieder eines fünf- bis siebengliedrigen Ringes, der aromatisch oder teilhydriert sein und bis zu 2 Heteroatome aus der Reihe O, N oder S enthalten kann,
$U^1$ Wasserstoff, Alkyl, Alkenyl, Cycloalkyl, Aralkyl, Aryl, Hydroxy, Alkoxy, Halogen, Dialkylamino, Nitro, Cyano, Alkylthio, Alkoxycarbonyl, Dialkylaminocarbonyl, Alkoxycarbonyloxy oder Alkylsulfonyl oder zusammen mit $R^1$ eine $C_2$- oder $C_3$-Brücke,

$V^1$ und $V^2$ unabhängig voneinander Wasserstoff, Alkyl, Alkenyl, Cycloalkyl, ggf. über Alkyl gebundene Aryl- oder heterocyclische Reste, -CO-$Z^1$,

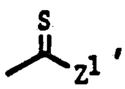, 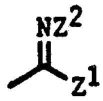,

CN oder gemeinsam mit dem stickstoff einen ggf. vollständig oder teilweise hydrierten, 0-3 Heteroatome der Reihe O, N, S, $SO_2$ enthaltenden Ring,
W Wasserstoff, Alkyl, Alkenyl, Cycloalkyl, ggf. über Alkyl gebundene Aryl- oder heterocyclische Reste,

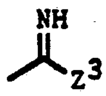

oder CN,
X Alkyl, Alkenyl, Cycloalkyl oder ggf. über Alkyl gebundene Aryl- oder heterocyclische Reste,
L Wasserstoff, Alkyl, Alkenyl, Cycloalkyl, Halogen, über Alkyl gebundene Aryl- oder heterocyclische Reste, oder die Bedeutung von $Y^1$ bzw. $Y^2$ hat,
$Y^1$ und $Y^2$ -CO-$Z^1$,

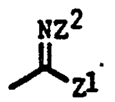,

-CN, -$SO_2$-$Z^1$, $NO_2$, Aryl oder ein heterocyclischer Rest,
$Z^1$ bis $Z^2$ Wasserstoff, Alkyl, Alkenyl, Cycloalkyl, Alkoxy, Alkylthio, Cycloalkoxy, Amino, Mono- oder Bisalkylamino, Arylamino oder ggf. über Alkyl oder Alkoxy gebundene Aryl-oder heterocyclische Reste oder $Z^1$ und $Z^2$ mit $V^1$ oder $V^2$ bzw. $Y^1$ oder $Y^2$ oder einem anderen Rest $Z^{1'}$ oder $Z^{2'}$ die fehlenden Glieder eines ggf. vollständig oder teilweise hydrierten, 0-2 Heteroatome der Reihe O, N, S oder $SO_2$ enthaltenden, ggf. benzanellierten Rings, wobei
$Z_1'$ und $Z^{2'}$ die Bedeutung von $Z^1$ bzw. $X^2$ besitzen und
n 1 oder 2 bedeuten
und alle cyclischen und nichtcyclischen Reste in der Farbstoffchemie übliche nichtionische Substituenten tragen können.

2. Tetraindolylheptamethinderivate gemäß Anspruch 1 der Formeln

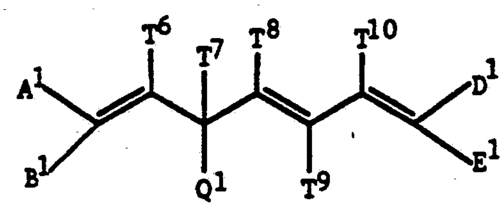

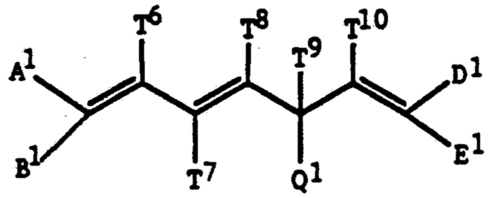

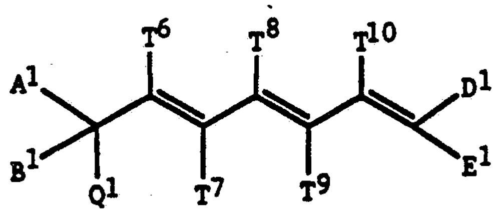

und

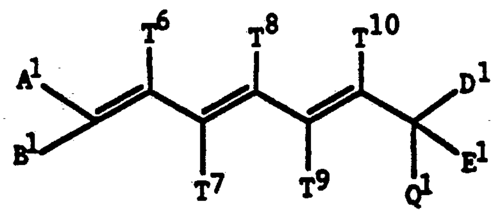

worin $A^1$, $B^1$, $D^1$ und $E^1$

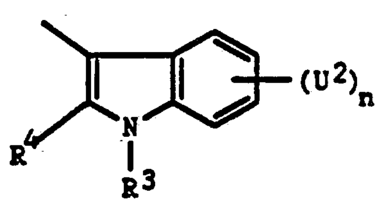

bedeuten und untereinander gleich oder verschieden sein können,

$Q^1$ $-NV^3V^4$, 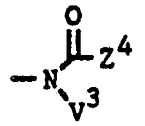, 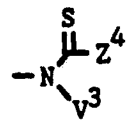, 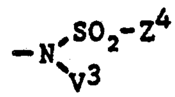,

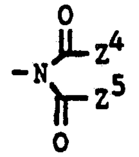, 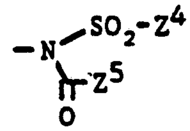, 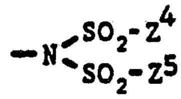, $-SW^1$,

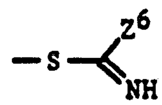, $-SO_2X^1$, 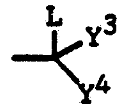, 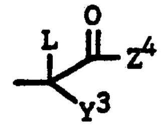,

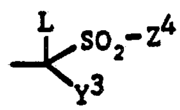, 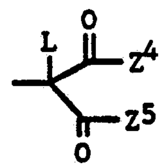, 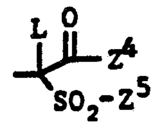, 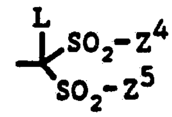

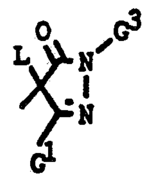, 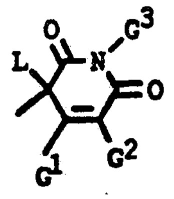,

$R^3$ Wasserstoff, $C_1$-$C_{18}$-Alkyl, das durch Chlor, $C_1$-$C_4$-Alkoxy, Cyano oder $C_1$-$C_4$-Alkoxycarbonyl substituiert sein kann, Allyl, Cyclopentyl, Cycloheyl, ggf. durch $C_1$-$C_4$-Alkyl, Chlor und/oder $C_1$-$C_4$-Alkoxy substituierte Benzyl-, Phenethyl-, Naphthylmethyl- oder Picolylreste,

$R^4$ Wasserstoff, $C_1$-$C_{18}$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, ggf. durch $C_1$-$C_4$-Alkyl, Chlor, Brom, $C_1$-$C_4$-Alkoxy, Cyano, Nitro und/oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl- oder Chinolylreste,

$T^6$ - $T^{10}$ Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Vinyl, Allyl, Cyclopentyl, cyclohexyl, Fluor, Chlor, Brom, $C_1$-$C_8$-Alkoxy, das noch durch $C_1$-$C_4$-Alkoxy substituiert sein kann, $C_1$-$C_4$-Dialkylamino, Piperidino, Pyrrolidino, Nitro, Cyano, $C_1$-$C_4$-Alkoxycarbonyl, ggf. durch $C_1$-$C_4$-Alkyl, Chlor, $C_1$-$C_4$-Alkoxy, $C_1$-$C_4$-Alkyl-sulfonyl, cyano und/oder $C_1$-$C_4$-Alkoxycarbonyl substituierte Benzyl-, Phenethyl-, Naphthylmethyl-, Picolyl-, Phenyl-, Naphthyl-, Pyridyl-, Chinolyl-, Pyrimidyl-, Pyrazinyl-, Indolyl-, Indolenyl-, Indolizinyl-, Imidazolyl-, Oxazolyl-, Thiazolyl-, Triazolyl-, Benzimidazolyl-, Benzoxazolyl-oder Benzthiazolylreste oder jeweils zwei der Reste $T^6$ bis $T^{10}$ eine Brücke der Formeln $-CH_2CH_2-$, $-CH_2CH_2CH_2-$, $-CH_2CH_2CH_2-$, $-CH_2SCH_2-$,

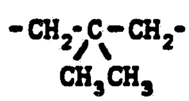, 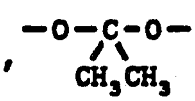,

$-CH=CH-CH_2-$, $-CH=CH-CH=CH-$,

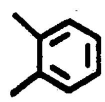, 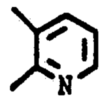,

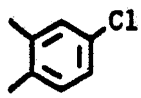,

$U^2$ Wasserstoff, $C_1$-$C_8$-Alkyl, Allyl, Cyclohexyl, Benzyl, Phenyl, Hydroxy, $C_1$-$C_4$-Alkoxy, Chlor, Brom, $C_1$-$C_4$-Dialkylamino, Nitro, Cyano, $C_1$-$C_4$-Alkylthio, $C_1$-$C_4$-Alkoxycarbonyl, $C_1$-$C_4$-Dialkylaminocarbonyl, $C_1$-$C_4$-Alkoxy-carbonyloxy oder $C_1$-$C_4$-Alkylsulfonyl oder zusammen mit $R^3$ eine $-CH_2CH_2-$ oder $-CH_2CH_2CH_2-$-Brücke, die durch maximal drei Methylgruppen substituiert sein kann,

$V^3$, $V^4$ und $W^1$ unabhängig voneinander Wasserstoff, $C_1$-$C_{18}$-Alkyl, das durch Chlor, $C_1$-$C_4$-Alkoxy, Cyano oder $C_1$-$C_4$-Alkoxycarbonyl substiuiert sein kann, Allyl, Cyclopentyl, Cyclohexyl, Cyano, ggf. durch $C_1$-$C_4$-Alkyl, Chlor, Brom, $C_1$-$C_4$-Alkoxy, Cyano, Nitro oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl-, Chinolyl-, Tetrahydrothiophendioxid-3-yl-, Piperidin-4-yl- oder Piperazin-1-yl-ethyl-Reste oder

$V^3$ und $V^4$ zusammen mit dem Stickstoff ggf. durch $C_1$-$C_4$-Alkyl, Chlor, $C_1$-$C_4$-Alkoxy oder Phenyl substituierte Pyrrolidin-, Piperidin-, Piperazin-, Morpholin-, Pyrazolin-, Pyrazol-, Imidazol-, Triazol- oder Tetrazol-Reste,

$X^1$ $C_1$-$C_{18}$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, ggf. durch $C_1$-$C_4$-Alkyl, Chlor, Brom, $C_1$-$C_4$-Alkoxy, Cyano, Nitro oder $C_1$-$C_4$-Alkoxycarbonyl substituierte Benzyl-, Phenethyl-, Naphthylmethyl-, Picolyl-, Chinolylmethyl-, Phenyl-, Naphthyl-, Pyridyl-, Pyrimidyl-, Pyrazinyl, Imidazolyl-, Oxazolyl-, Thiazolyl-, Triazolyl-, Benzimidazolyl-, Benzoxazolyl-, Benzthiazolyl- oder Chinolyl-Reste,

L Wasserstoff, $C_1$-$C_8$-Alkyl, das durch chlor, $C_1$-$C_4$-Alkoxy, Cyano oder $C_1$-$C_4$-Alkoxycarbonyl substituiert sein kann, Allyl, Cyclopentyl, Cyclohexyl, Chlor, Cyano, $C_1$-$C_8$-Alkoxycarbonyl, ggf. durch $C_1$-$C_4$-Alkyl, Chlor, $C_1$-$C_4$-Alkoxy, Cyano, Nitro oder $C_1$- $C_4$-Alkoxycarbonyl substituierte Benzyl-, Phenethyl-, Picolyl-, Phenyl-, Naphthyl-oder Pyridyl-Reste,

$Y^3$ und $Y^4$ Cyano, Nitro oder ggf. durch $C_1$-$C_4$-Alkyl, Chlor, Brom $C_1$-$C_4$-Alkoxy, Cyano, Nitro, $C_1$-$C_4$-Alkylsulfonyl oder $C_1$-$C_4$-Alkoxycarbonyl substituierte Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl-oder Chinolyl-Reste,

$Z^4$,$Z^5$,$Z^6$ unabhängig voneinander Wasserstoff $C_1$-$C_{18}$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, $C_1$-$C_{18}$-Alkoxy, $C_1$-$C_{18}$-Alkylthio, Cyclopentoxy, Cyclohexoxy, Amino, mono- oder bis-$C_1$-$C_{12}$-Alkylamino, ggf. durch $C_1$-$C_4$-Alkyl, Chlor, Brom, $C_1$-$C_4$-Alkoxy, Cyano, Nitro oder $C_1$-$C_4$- Alkoxycarbonyl substituierte Anilino-, N-$C_1$-$C_4$-Alkylanilino-, Diphenylamino-, Benzyl, Benzyloxy-, Benzylamino-, N-$C_1$-$C_4$-Alkylbenzylamino, Dibenzylamino-, Phenethyl-, Phenethoxy-, Phenethamino-, Pyridylethoxy-, Pyridylamino-, Picolyl-, Phenyl-, Naphthyl-, Pyridyl-, Pyrimidyl-, Pyrazinyl-, Imidazolyl-, Oxazolyl-, Thiazolyl-, Triazolyl-, Benzimidazolyl-, Benzoxazolyl-, Benzthiazolyl-, Chinolyl- oder Piperazin-1-yl-ethyl-Reste oder

$Z^4$ mit $Z^5$ oder $V^3$ oder $Y^3$ eine Brücke der Formeln $-CH_2CH_2-$, $-CH=CH-$, $-CH_2CH_2CH_2-$, $-CH_2CH_2CH_2CH_2-$,

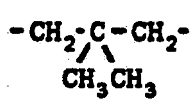, 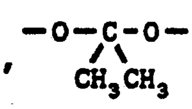,

$-CH=CH-CH_2-$, $-CH=CH-CH=CH-$,

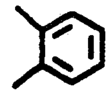,

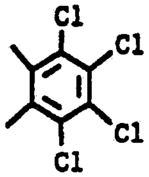, 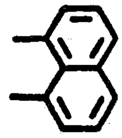, 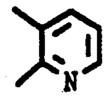, 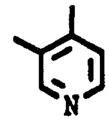,

$G^1$ und $G^2$ unabhängig voneinander Wasserstoff, $C_1$-$C_8$-Alkyl, das durch Chlor, $C_1$-$C_4$-Alkoxy, Cyano oder $C_1$-$C_4$-Alkoxycarbonyl substituiert sein kann, Allyl, Cyclopentyl, Cyclohexyl, Hydroxy, Chlor, $C_1$-$C_8$-Alkoxy, Cyano, $C_1$-$C_8$-Alkoxycarbonyl, Nitro, $C_1$-$C_8$-Alkylsulfonyl, ggf. durch $C_1$-$C_4$-Alkyl, Chlor, $C_1$-$C_4$-Alkoxy, cyano, Nitro oder $C_1$-$C_4$-Alkoxycarbonyl substituierte Benzyl-, Phenethyl-, Picolyl-, Phenyl-, Napthyl-, Pyridyl-, Imidazolyl-, Oxazolyl-, Thiazolyl-, Triazolyl-, Benzimidazolyl-, Benzthiazolyl- oder Benzoxazolyl-Reste,

$G^3$ Wasserstoff, $C_1$-$C_8$-Alkyl, das durch chlor, $C_1$-$C_4$-Alkoxy, Cyano oder $C_1$-$C_4$-Alkoxycarbonyl substituiert sein kann, allyl, Cyclopentyl, Cyclohexyl, ggf. durch $C_1$-$C_4$-Alkyl, Chlor, $C_1$-$C_4$-Alkoxy, Cyano, Nitro oder $C_1$- $C_4$-Alkoxycarbonyl substituierte Benzyl-, Phenethyl-, Picolyl-, Phenyl-, Naphthyl-oder Pyridyl-Reste und n 1 oder 2 bedeuten.

3. Tetraindolylheptamethinderivate der Formeln des Anspruchs 2, worin

$Q^1$ $C_1$-$C_8$-Mono- oder Dialkylamino, ggf. durch Methyl, Chlor oder Methoxy substituierte Pyrrolidino-, Piperidino-, Morpholino-, Piperazino-, Hydroxyethylpiperazino-, Pyrazolo-, Imidazolo-, Triazolo-, Tetrazolo-Reste, ggf. durch Methyl, Ethyl, Chlor, Cyano oder Nitro substituierte Anilino-, N-$C_1$-$C_4$-Alkylanilino-, Diphenylamino-, Benzylamino-, N-$C_1$-$C_4$-Alkyl-N-benzylamino-, Pyridylamino-, N-Phenylbenzoylamino-, Benzolsulfonylamino-, N-$C_1$-$C_4$-Alkylbenzolsulfonylamino-, Phthalimido-, Naphthalimido-, Homophthalimido-, Benzoesäure-2-sulfonsäureimido-, Succinimido-, Maleinimido-Reste, Aminocarbonylamino, $C_1$-$C_8$-Alkoxycarbonylamino, $C_1$-$C_8$-Alkylthio, ggf. durch Methyl, Chlor oder Methoxy substituiertes Phenyl thio, $H_2N\text{-}\underset{\underset{NH}{\|}}{C}\text{-}S\text{-}$,

$C_1$-$C_8$-Alkylsulfonyl, ggf. durch Methyl, ethyl, Chlor, Brom, Cyano oder Methoxycarbonyl substituierte Phenylsulfonyl-, Benzylsulfonyl-, Naphthylsul fonyl-, in $\alpha$-Stellung durch cyano, $C_1$-$C_4$-Alkoxycarbonyl, $C_1$-$C_4$-Alkylsulfonyl, $C_1$-$C_4$-Alkanoyl oder Benzoyl substituierte, ggf. weiterhin durch Methyl, Ethyl, Chlor, Brom, Cyano, Nitro oder Methoxycarbonyl substituierte Benzyl-, Naphthylmethyl-, Picolyl-, Chinolylmethyl-, Imidazolylmethyl-, Benzimidazolylmethyl-, Oxazolylmethyl-, Benzoxazolylmethyl-, Thiazolylmethyl-, Benzthiazolylmethyl-, Triazolylmethyl-, Tetrazolylmethyl-Reste, Dicyanomethyl, Cyano-$C_1$-$C_4$-Alkoxycarbonylmethyl, Bis-$C_1$-$C_4$-Alkoxy-carbonylmethyl, Cyano-$C_1$-$C_4$-alkanoylmethyl, Bis-$C_1$-$C_4$-alkanoylmethyl, ggf. durch Methyl, Ethyl, Chlor, Cyano, Nitro oder Methoxycarbonyl substituierte Cyano-benzoylmethyl-, $C_1$-$C_4$-Alkanoyl-benzoylmethyl-, $C_1$-$C_4$-Alkoxycarbonyl-benzoylmethyl-, Dibenzoylmethyl-, Cyano-phenylsulfonylmethyl-, $C_1$-$C_4$-Alkanoyl-phenylsulfonylmethyl-, $C_1$-$C_4$-Alkoxycarbonl-phenylsulfonylmethyl-, Bisphenylsulfonylmethyl-Reste, die Reste von ggf. durch Methyl, Ethyl, Chlor, Methoxy, Ethoxy, Phenyl, Cyano, Methoxy- oder Ethoxycarbonyl substituiertem 1,3-Cyclopentandion, 1,3-Cyclohexandion, 1,3-Indandion, Meldrumsäure, Pyrazolon, 2-Hydroxy-6-pyridon oder Barbitursäure,

$R^3$ Wasserstoff, $C_1$-$C_8$-Alkyl, das durch chlor, Methoxy, Ethoxy, Cyano oder Methoxycarbonyl substituiert sein kann, Allyl, Cyclopentyl, Cyclohexyl, ggf. durch Methyl, Chlor oder Methoxy substituierte Benzyl-, Phenethyl-oder Picolyl-Reste,

$R^4$ Wasserstoff, $C_1$-$C_8$-Alkyl, das durch Chlor, Methoxy, Ethoxy, Cyano oder Methoxycarbonyl substituiert sein kann, Allyl, Cyclopentyl, Cyclohexyl, ggf. durch Methyl, Chlor, Methoxy, Cyano, Nitro und/oder Methoxycarbonyl substituerte Benzyl-, Phenethyl-, Picolyl-, Phenyl, Naphthyl-, Pyridyl-, Pyrimidyl-, Benzimidazolyl-, Benzoxazolyl-, Benzthiazolyl- oder Chinolyl-Reste,

$T^6$ u. $T^{10}$ Wasserstoff, $C_1$-$C_8$-Alkyl, das durch Chlor, Methoxy, Cyano oder Methoxycarbonyl substituiert sein kann, Vinyl, Allyl, Cyclopentyl, Cyclohexyl, Chlor, $C_1$-$C_8$-Alkoxy, Cyano, Methoxycarbonyl, Nitro, Benzyl, ggf. durch Methyl, Chlor, Cyano oder Methoxy substituierte Phenyl- oder Pyridyl-Reste,

$T^7$ bis $T^9$ Wasserstoff, $C_1$-$C_8$-Alkyl, das durch chlor, Methoxy, Cyano oder Methoxycarbonyl substituiert sein kann, Allyl, Cyclopentyl, Cyclohexyl, Chlor, Brom, Cyano, Methoxy-oder Ethoxycarbonyl, Nitro, $C_1$-$C_4$-Alkoxy, $C_1$-$C_4$-Dialkylamino, Benzyl, ggf. durch Methyl, ethyl, Chlor, Methoxy, Ethoxy, Cyano, Nitro und/oder Methoxycarbonyl substituierte Phenyl-, Naphthyl-, Pyridyl-, Chinolyl-, Pyrimidyl-, Indolenyl-, Indolizinyl-, Imidazolyl-, Oxazolyl-, Thiazolyl-, Benzimidazolyl-, Benzoxazolyl- oder Benzthiazolyl-Reste oder

$T^7$ mit $T^8$ oder $T^9$ oder $T^8$ mit $T^9$ eine Brüce der Formeln $-Ch_2CH_2-$, $-CH_2CH_2CH_2-$,

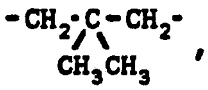 ,

$-CH=CH-CH_2-$, $-CH=CH-CH=CH-$ oder

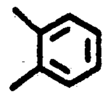 ,

$U^2$ Wasserstoff, $C_1$-$C_4$-Alkyl, Cyclohexyl, Benzyl, $C_1$-$C_4$-Alkoxy, Chlor, $C_1$-$C_4$-Dialkylamino, Nitro, Cyano, Methoxy- oder Ethoxycarbonyl oder Methylsulfonyl, wobei $U^2$ in 5-, 6- und/oder 7-Postition am Indolylrest stehen kann, oder ein in 7-Postion stehender Rest $U^2$ zusammen mit $R^3$ eine Brücke der Formeln

$-CH_2CH_2-$, 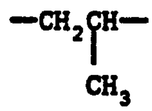 , 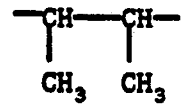 ,

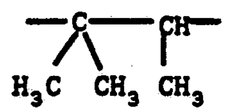 , $-CH_2CH_2CH_2-$, 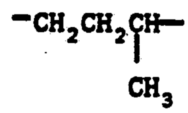 ,

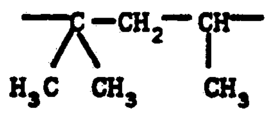

bilden kann, und

n 1 oder 2 bedeuten.

4. Tetraindolylheptamethinderivate der Formel

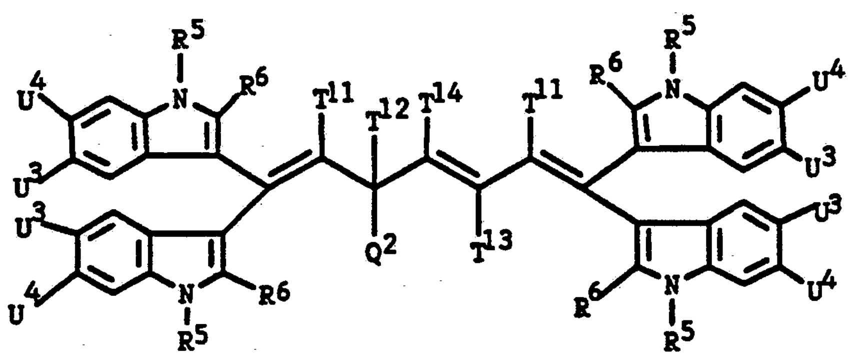

und ihre bezüglich der Stellung der $Q^2$-Gruppe isomeren Formen, wie sie in Anspruch 1 wiedergegeben sind, worin

$Q^2$ Dimethylamino, Diethylamino, Dipropylamino, Dibutylamino, Propylamino, Butylamino, Pyrrolidino, Piperidino, Morpholino, Piperazino, Pyrazolo, Imidazolo, Triazolo, Anilino, 4-Methylanilino, 4-Chloranilino, 4-Methoxyanilino, 4-Nitroanilino, N-Methylanilino, Benzylamino, N-Methylbenzylamino, N-Ethylbenzylamino, Benzoylamino, Benzolsulfonylamino, Phthalimido, Naphthalimido, Succinimido, Maleinimido, Methylthio, Ethylthio, Phenylthio, Methylsulfonyl, Ethylsulfonyl, Propylsulfonyl, Butylsulfonyl, Hexylsulfonyl, Octylsulfonyl, Benzolsulfonyl, 4-Methylbenzolsulfonyl, 4-Chlorbenzolsulfonyl, α-Cyanobenzyl, α-Cyano-4-methylbenzyl, α-Cyano-4-chlorbenzyl, α-Cyano-4-nitrobenzyl, α-Cyano-α-pico lyl, α-Cyano-γ-picolyl, Dicyanomethyl, Cyano-methoxycarbonylmethyl, Cyano-ethoxy-carbonylmethyl, Bis-methoxycarbonylmethyl, Bis-ethoxycarbonylmethyl, 1-Cyanoaceton-1-yl, 1-Cyanobutanon-1-yl, 1-Methoxycarbonylaceton-1-yl, 1-Ethoxycarbonylaceton-1-yl, 1,3-Pentandion-3-yl, 1-Benzoylaceton-1-yl, 2-Cyano-1-phenylethanon-2-yl, Dibenzoylmethyl, Cyano-phenylsulfonylmethyl, Bisphenylsulfonylmethyl, 1,3-Cyclopentandion-2-yl, 1,3-Cyclohexandion-2-yl, 5,5-Dimethyl-1,3-cyclohexandion-2-yl, 1,3-Indandion-2-yl, 2,2-Dimethylperhydro-1,3-dioxin-4,6-dion-5-yl, 3-Methyl-1-phenyl-2-pyrazolin-5-on-4-yl, 1-(2-Chlorphenyl)3-methyl-2-pyrazolin-5on-4-yl, 3-Methyl-1-(4-methylphenyl)-2-pyrazolin-5-on-4-yl, 3-Methyl-1-(4-nitrophenyl)-2-pyrazolin-5-on-4-yl, 3-Methyl-1-phenyl-5-imino-2-pyrazolin-4-yl, 3-Methyl-1-(3-sulfolanyl)-2-pyrazolin-5-on-4-yl, 3-Ethoxycarbonyl-1-phenyl-2-pyrazolin-5-on-4-yl, 3-Aminocarbonyl-1-phenyl-2-pyrazolin-5-on-4-yl, 4-Hydroxy-1-methyl-2(1H)-chinolinon-3-yl, 3-Cyano-4-methyl-6-hydroxy-2(1H)-pyridon-5-yl, 3-Cyano-1,4-dimethyl-6-hydroxy-2(1H)-pyridon-5-yl, 2,4,6-(1H,3H,5H)-Pyrimidintrion-5-yl,

$R^5$ Methyl, Ethyl, Propyl, Butyl, Hexyl, Octyl, 2-Cyanoethyl, 2-Methoxyethyl, 2-Methoxycar bonylethyl, 2-Chlorethyl, 2-Acetoxyethyl, Cyclohexyl, Allyl oder Benzyl,

$R^6$ Methyl, Ethyl, Propyl, Butyl, Hexyl, Octyl, Cyclohexyl, Benzyl, Phenyl, 2-, 3- oder 4-Chlorphenyl, 2-, 3- oder 4-Methoxyphenyl, 4-Nitrophenyl, 2,4-Dichlorphenyl, 2-, 3-oder 4-Tolyl oder 2-, 3- oder 4-Pyridyl,

$T^{11}$ Wasserstoff, Methyl, Ethyl, Propyl, Butyl, Vinyl, 2-Chlorethyl, 2-Cyanoethyl, Chlor, Cyano, Phenyl, 4-Tolyl oder 4-Chlorphenyl,

$T^{12}$, $T^{13}$ Wasserstoff, Methyl, Ethyl, Propyl, Butyl, Chlor, Cyano, Methoxycarbonyl, Dimethylamino, Phenyl, 4-Tolyl, 4-Chlorphenyl, Pyridyl oder $T^{12}$ und $T^{13}$ gemeinsam eine Gruppierung der Formeln $-CH_2CH_2-$, $-CH_2CH_2CH_2-$,

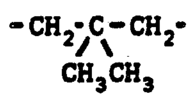 , 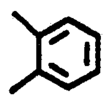 ,

$T^{14}$ Wasserstoff, Methyl, Ethyl, Propyl, Butyl, Chlor, Brom, Cyano, Phenyl, 4-Tolyl, 4-Chlorphenyl, 4-Nitrophenyl, 4-Pyridyl, 3,3-Dimethyl-indolen-2-yl, Indolizin-2-yl, 2-Benzimidazolyl, 2-Benzoxazolyl oder 2-Benzthiazolyl,

$U^3$ und $U^4$ Wasserstoff, Methyl, Methoxy, Chlor, Cyano, Methoxycarbonyl oder Nitro bedeuten.

5. Verfahren zur Herstellung von Tetraindolylheptamethinderivaten des Anspruchs 1, dadurch gekennzeichnet, daß man Ethene der Formeln

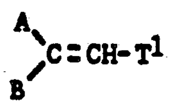

und/oder

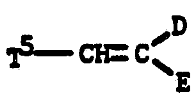

oder Carbinole der Formeln

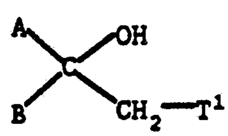

und/oder

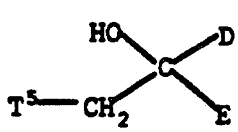

oder Salze der Formeln

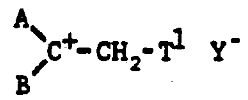

und/oder

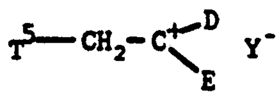

mit 1,3-Dicarbonylverbindungen der Formel

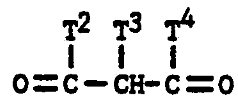

oder deren Acetale oder Ketale der Formel

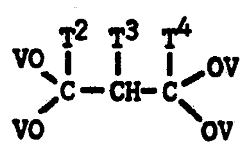

oder vinylogen Amidiniumsalze der Formel

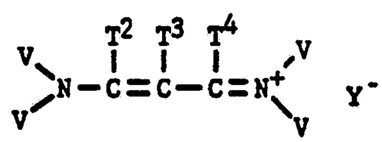

oder vinylogen Chlorimmoniumsalze der Formeln

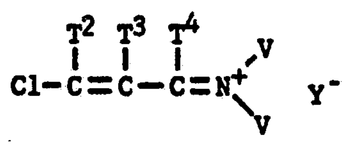

oder

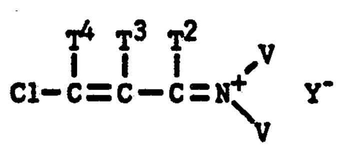

kondensiert und mit Verbindungen der Formel
HQ
umsetzt, worin
A, B, D, E, Q und $T^1$ bis $T^5$ die in Anspruch 1 angegebene Bedeutung besitzen und
V für Alkyl, Aralkyl oder Aryl und
$Y^-$ für ein Anion stehen.

6. Verfahren zur Herstellung von Tetraindolylheptamethinderivaten des Anspruchs 1, dadurch gekennzeichnet, daß man Tetraindolylheptamethinether oder -alkohole der isomeren Formeln

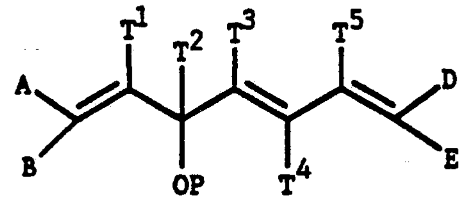

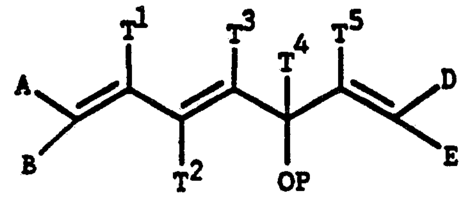

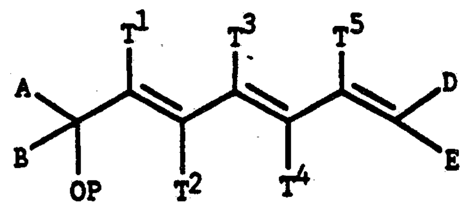

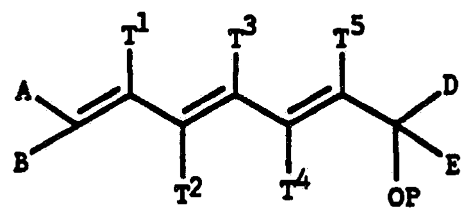

mit HQ umsetst, worin
A, B, D, E, Q und $T^1$ bis $T^5$ die in Anspruch 1 angegebene Bedeutung besitzen und
P Wasserstoff, Alkyl, Alkenyl, Cycloalkyl, Aralkyl, Aryl oder einen über Alkyl gebundenen heterocyclischen Rest bedeutet.

7. Mischungen von Tetraindolylheptamethinderivaten, dadurch gekennzeichnet, daß sie nach dem Verfahren des Anspruchs 5 hergestellt werden.

8 Verwendung der Tetraindolylheptamethinderivate des Anspruchs 1 für druck- oder thermoempfindliche Aufzeichnungsmaterialien.

9. Druck- oder thermoempfindliches Aufzeichnungsmaterial, dadurch gekennzeichnet, daß es Tetraindolylheptamethinderivate des Anspruchs 1 enthält.

Europäisches Patentamt

# EUROPÄISCHER RECHERCHENBERICHT

Nummer der Anmeldung

EP 89117618.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 951 353 (BASF) * Tabelle 5; Beispiele 63,69; Ansprüche 1,2 * -- | 1,8 | C 07 D 209/10<br>C 07 D 403/06<br>B 41 M 5/124 |
| A | DE - A1 - 2 265 233 (CIBA-GEIGY) * Ansprüche 1,7,8 * -- | 1,8,9 | |
| A | DE - B1 - 1 248 193 (BASF) * Beispiel 2 * -- | 1 | |
| A | DE - A1 - 2 257 711 (CIBA-GEIGY) * Ansprüche 1,12,19,22-26 * -- | 1,8,9 | |
| A | CHEMICAL ABSTRACTS, Band 107, Nr. 13, 18. September 1987, Columbus, Ohio, USA BANERJI, J. et al. "Electrophilic substitution of indoles. Part IX. Reaction of indoles with ininium systems" Seite 615, Spalte 1, Zusammenfassung-Nr. 115 454w & Indian J. Chem., Sect. B 1986, 25B(12), 1204-8 -- | 1 | RECHERCHIERTE SACHGEBIETE (Int. Cl.4)<br>C 07 D 209/00<br>C 07 D 403/00<br>B 41 M 5/00 |
| A | CHEMICAL ABSTRACTS, Band 101, Nr. 24, 10. Dezember 1984, Columbus, Ohio, USA KONYUKHOVA, T.M. et al. "3,3-Bis(3-indolyl)phthalides and their properties" Seite 66, Spalte 2, Zusammenfassung-Nr. 212 655w & Zh. Vses. Khim. O-va.im. D.I. Mendeleeva 1984, | 1 | |

Der vorliegende Recherchenbericht wurde für alle Patentansprüche erstellt.

| Recherchenort | Abschlußdatum der Recherche | Prufer |
|---|---|---|
| WIEN | 03-01-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 89117618.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | 29(4), 467-8<br>-- | | |
| A | CHEMICAL ABSTRACTS, Band 89, Nr. 13, 25. September 1978, Columbus, Ohio, USA<br>BERTI, C.. et al. "Synthesis and reactivity of indolylmethyl cations"<br>Seite 872, Spalte 2, Zusammenfassung-Nr. 109 002r<br>& J. Heterocycl. Chem. 1978, 15(3), 433-7<br>-- | 1 | |
| A | CHEMICAL ABSTRACTS, Band 70, Nr. 22, 2. Juni 1969, Columbus, Ohio, USA<br>LIN, CHAO-HAN "3,3-Bis(indol--3-yl)phthalides"<br>Seite 74, Spalte 1, Zusammenfassung-Nr. 97 960u<br>& S. African 68 00,323<br>-- | 1 | RECHERCHIERTE SACHGEBIETE (Int. Cl.4) |
| A | CHEMICAL ABSTRACTS, Band 93, Nr. 5, 4. August 1980, Columbus, Ohio, USA<br>VOLOVEL'SKII, L.N. et al. "Indole derivatives of steroid ketones"<br>Seite 966, Spalte 2, Zusammenfassung-Nr. 46 967u<br>& Zh. Obshch. Khim. 1980, 50(1), 190-5<br>---- | 1 | |

Der vorliegende Recherchenbericht wurde für alle Patentansprüche erstellt.

| Recherchenort | Abschlußdatum der Recherche | Prüfer |
|---|---|---|
| WIEN | 03-01-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82